(19) Europäisches Patentamt

European Patent Office

Office européen des brevets

(11) **EP 1 386 973 B1**

(12) **EUROPEAN PATENT SPECIFICATION**

(45) Date of publication and mention
of the grant of the patent:
**09.08.2006 Bulletin 2006/32**

(51) Int Cl.:
*C12Q 1/68* [(2006.01)]      *C12N 15/09* [(2006.01)]
*G06F 19/00* [(2006.01)]

(21) Application number: **02717173.5**

(22) Date of filing: **16.04.2002**

(86) International application number:
**PCT/JP2002/003770**

(87) International publication number:
**WO 2002/086161 (31.10.2002 Gazette 2002/44)**

(54) **METHOD OF INFERRING DIPLOTYPE CONFIGURATION FROM THE GENOTYPE OF INDIVIDUAL**

VERFAHREN ZUR ABSCHÄTZUNG DES DIPLOTYPS AUS DEM GENOTYP EINES INDIVIDUUMS

PROCEDE PERMETTANT D'EVALUER LE DIPLOTYPE DU GENOTYPE D'UN INDIVIDU

(84) Designated Contracting States:
**AT BE CH CY DE DK ES FI FR GB GR IE IT LI LU
MC NL PT SE TR**

(30) Priority: **19.04.2001 JP 2001121641**

(43) Date of publication of application:
**04.02.2004 Bulletin 2004/06**

(73) Proprietors:
• **Hubit Genomix, Inc.
Tokyo 102-0092 (JP)**
• **StaGen Co., Ltd.
Chiba 272-0035 (JP)**

(72) Inventors:
• **KAMATANI, Naoyuki
Shibuya-ku, Tokyo 150-0002 (JP)**
• **KITAMURA, Yutaka
Koto-ku, Tokyo 136-0076 (JP)**

(74) Representative: **Warcoin, Jacques et al
Cabinet Régimbeau,
20, rue de Chazelles
75847 Paris Cedex 17 (FR)**

(56) References cited:
**WO-A-01/80156      WO-A-01/91026
WO-A2-99/52942**

• ROHDE K ET AL: "HAPLOTYPING AND
ESTIMATION OF HAPLOTYPE FREQUENCIES
FOR CLOSELY LINKED BIALLELIC
MULTILOCUS GENETIC PHENOTYPES
INCLUDING NUCLEAR FAMILY INFORMATION"
HUMAN MUTATION, WILEY-LISS, NEW YORK,
NY, US, vol. 17, no. 4, 2 April 2001 (2001-04-02),
pages 289-295, XP009026283 ISSN: 1059-7794
• GHOSH SAURABH ET AL: "Mapping a
quantitative trait locus via the EM algorithm and
Bayesian classification" GENETIC
EPIDEMIOLOGY, vol. 19, no. 2, September 2000
(2000-09), pages 97-126, XP009032795 ISSN:
0741-0395
• KUHNER M K ET AL: "Maximum likelihood
estimation of recombination rates from
population data." GENETICS. UNITED STATES
NOV 2000, vol. 156, no. 3, November 2000
(2000-11), pages 1393-1401, XP002285940 ISSN:
0016-6731
• ADAM G IR ET AL: "Pharmacogenomics to
predict drug response" PHARMACOGENOMICS,
ASHLEY PUBLICATIONS, GB, vol. 1, no. 1, 2000,
pages 5-14, XP002961165 ISSN: 1462-2416
• JOHNSON D L ET AL: "MAPPING QUANTITATIVE
TRAIT LOCI IN A SELECTIVELY GENOTYPED
OUTBRED POPULATION USING A MIXTURE
MODEL APPROACH" GENETICAL RESEARCH,
CAMBRIDGE UNIVERSITY PRESS, CAMBRIDGE,
GB, vol. 73, no. 1, February 1999 (1999-02), pages
75-83, XP001070121 ISSN: 0016-6723

**EP 1 386 973 B1**

- KITAMURA Y ET AL: "Determination of probability distribution of diplotype configuration (diplotype distribution) for each subject from genotypic data using the EM algorithm." ANNALS OF HUMAN GENETICS. ENGLAND MAY 2002, vol. 66, no. Pt 3, May 2002 (2002-05), pages 183-193, XP009032701 ISSN: 0003-4800
- FALLIN D. ET AL.: 'Accuracy of haplotype frequency estimation for biallelic loci, via the expectation-maximization algorithm for unphased diploid genotype data' AM. J. HUM. GENET. vol. 67, 2000, pages 947 - 959, XP002953525
- NIELSEN D.M. ET AL.: 'Detecting marker-disease association by testing for Hardy-Weinberg disequilibrium at a marker locus' AM. J. HUM. GENET. vol. 63, 1999, pages 1531 - 1540, XP002953526
- OSIER M. ET AL.: 'Linkage disequilibrium at the ADH2 and ADH3 loci and risk of alcoholism' AM. J. HUM. GENET. vol. 64, 1999, pages 1147 - 1157, XP002953527
- LONG J.C. ET AL.: 'An E-M algorithm and testing strategy for multiple-locus haplotypes' AM. J. HUM. GENET. vol. 56, 1995, pages 799 - 810, XP002944464
- EXCOFIER L. ET AL.: 'Maximum-likelihood estimation of molecular haplotype frequencies in a diploid population' MOL. BIOL. EVOL. vol. 12, 1995, pages 921 - 927, XP002953528

**Description**

Technical Field

The present invention relates to methods and devices for inferring diplotype configurations from the genotype of an individual without family data, and uses thereof.

Background Art

Characteristics (such as susceptibility to a particular disease, drug response or side effects thereto) that are intrinsic to an individual, yet different among individuals and associated with genetic polymorphisms are referred to as "phenotypes". Normally, in any region of genes, polymorphic sites, such as multiple single nucleotide substitutions (SNP: single nucleotide polymorphism), microsatellites, insertions/deletions, exist proximally and are linked together to form haplotypes. Data regarding haplotypes are important for selecting markers that may be used in mapping disease-associated genes and for efficient computation of relative risk of an individual to develop a phenotype such as diseased.

Each individual carries two haplotypes for the genetic site on autosomes. A set of such haplotype pairs intrinsic to an individual is referred to as "diplotype configuration". Therefore, all information on a genetic region of an individual is carried by its diplotype configuration. The above-mentioned phenotype of an individual can be stochastically determined in accordance with the diplotype configuration. However, without familial data, analyses for genomic genes merely provide data on genotypes of multiple gene loci. The term "genotype" generally refers to a set of two polymorphisms, such as single nucleotide substitution, microsatellite and insertion/deletion at a single gene locus derived from the mother and the father, respectively. Generally obtained information on the genotypes of multiple-linked gene loci for an individual is incomplete but more accurate genetic information can be identified for an individual from its diplotype configuration. The phenotype of the individual is expected to be inferred more accurately based on such information. For example, an effect or side effect generated by a certain drug may be predicted based on the phenotype inferred, as described above. Alternatively, in cases of specific diseases wherein a subject cannot be determined to have the disease, an advantageous therapeutic method can be selected for the individual based on the inferred diplotype configuration.

Several methods for computing haplotype frequency in a population are known in the art. For example, analytical method (Elandt-Johnson, R.C. (1971) Probability models and statistical methods in genetics. New York, Wiley; Hill, W.G. (1974) Estimation of linkage disequilibrium in randomly mating populations. Heredity 33, 229-239; Yasuda, N. (1978) Estimation of haplotype frequency and linkage disequilibrium parameter in the HLA system. Tissue Antigena 12, 315-322; Imanishi, T., et al. (1991) Estimation of allele and haplotype frequencies for HLA and complement loci. In HLA: Proceedings of the Eleventh International Histocompatibility Workshop and Conference (eds. Tsuji, K., et al.), Vol.1, pp. 76-79, Oxford University Press, New York); sequential haplotype inference algorithm (Clark, A. G. (1990) Inference of haplotypes from PCR-amplified samples of diploid populations. Mol. Biol. Evol. 7, 111-122) and maximum-likelihood estimation procedure based on the expectation-maximization (EM) algorithm (Excoffier, L. & Slatkin, M. (1995) Maximum-likelihood estimation of molecular haplotype frequencies in a diploid population. Mol. Biol. Evol. 12, 921-927; Hawley, M. E. & Kidd, K. K. (1995) HAPLO: a program using the EM algorithm to estimate the frequencies of multi-site haplotypes. J. Heredity 86, 409-411; Long, J. C., et al. (1995) An E-M algorithm and testing strategy for multiple-locus haplotypes. Am. J. Hum. Genet. 56, 799-810; Fallin, D. & Schork, N. J. (2000) Accuracy of haplotype frequency estimation for biallelic loci, via the expectation-maximization algorithm for unphased diploid genotype data. Am. J. Hum. Genet. 67, 947-959).

However, it remains to be determined whether the haplotype frequency estimated by these methods is accurate. In addition, hitherto no method for inferring a diplotype configuration from the genotype of an individual has been reported. Thus, development of methods for efficiently inferring a diplotype configuration of an individual is desired in the art.

Disclosure of the Invention

The present invention was undertaken in view of such a background. Thus, an objective of the present invention is to provide methods and devices for inferring an individual's diplotype configuration from the genotype information without family data. Another objective of the present invention is to provide uses of the diplotype configuration inferred by these methods.

The present inventors undertook extensive studies to achieve the above-described objectives. First, the present inventors inferred diplotype configuration for each individual in a population and checked whether the inferred diplotype configuration agreed with the diplotype configuration determined in an *in-vitro* experiment. The present inventors determined the physical diplotype configuration for each individual by amplifying portions of smoothelin (SMTN) and serum amyloid A (SAA) genes by PCR, and analyzed the amplified segments by denaturing high-performance liquid chromatography (DHPLC) and single strand conformational polymorphism (SSCP). Then, the haplotype frequency for the population was computed based on the genotypic information of a number of linked genetic loci of each individual. The computation was conducted using the expectation-maximization (EM) algorithm. Based on the computed haplotype frequency and the genotypic information for each individual, the probability distribution of diplotype configuration (diplotype distribution) for each individual was determined and it was examined whether the distribution was centered in a single diplotype configuration. As a result, in most cases the diplotype distribution for each individual was found to center in a single diplotype configuration. However, the diplotype configurations for some individuals exhibited probability distributions. Next, thus determined diplotype configuration was compared with that determined in an *in-vitro* experiment. Surprisingly, the two-diplotype configurations were found to be identical to each other.

Thus, diplotype distribution of an individual could be computed by the use of haplotype frequency computed from the genotypic information of the individual. In other words, the diplotype configuration of an individual could be inferred from the genotypic information of the individual. Furthermore, the diplotype configuration inferred via this procedure is considered highly reliable.

The diplotype distribution computed according to this method will find diverse applications. For example, the probability of a subject to exhibit a specific phenotype can be computed based on the computed diplotype distribution and the previously estimated or determined probability of an individual with a specific diplotype configuration to exhibit a specific phenotype (penetrance). Alternatively, the probability whether a specific drug is effective or causes side effect in a subject can be computed based on the computed diplotype distribution and the previously estimated or determined probability of the specific drug to show effect or side effect in an individual with a specific diplotype configuration. Furthermore, the probability that a subject develops or is affected with a specific disease can also be computed based on the computed diplotype distribution and the previously estimated or determined probability that an individual having a specific diplotype configuration develops or is affected with the specific disease. The computed data of probabilities are useful, particularly for physicians to select an appropriate drug, an appropriate dose, or methods for treating or preventing a disease of an individual. In other words, the data can be employed to deliver tailor-made medical service.

In addition, data of haplotype frequency and diplotype distribution of a population obtained according to the method of the present invention, and databases constructed based on the data of diplotype configurations, are expected to serve as important information sources in conveniently and rapidly providing such tailor-made medical service.

Specifically, the present invention relates to methods and devices for inferring diplotype configuration from genotypic information of an individual without family data, and uses of the inferred diplotype configuration. More specifically, the present invention provides:

[1] a method for computing diplotype distribution for each subject in a population without family data, which comprises the step of computing the diplotype distribution for each.subject based on previously estimated or determined haplotype frequencies in the population and the genotypic information of each subject;

[2] the method according to [1], wherein the haplotype frequencies are computed in the population from genotypic information of each subject in the population just prior the step of computing the diplotype distribution;

[3] the method according to [2], wherein the computation of haplotype frequencies is performed using the EM algorithm;

[4] the method according to [1] or [2], wherein the computation of diplotype distribution is performed using Bayes' theorem or computation of posterior probability;

[5] the method according to any one of [1] to [4], wherein the genotypic information is a polymorphism selected from the group consisting of single nucleotide polymorphism (SNP), microsatellite polymorphism and insertion/deletion polymorphism;

[6] a method for computing the probability of a subject to exhibit a specific phenotype, which comprises the steps of:

(a) computing diplotype distribution for the subject without family data using the method according to any one of [1] to [5]; and

(b) computing the probability that the subject exhibits the specific phenotype from the diplotype distribution computed in step (a) based on previously estimated or determined probability that an individual having a specific diplotype configuration exhibits the specific phenotype;

[7] a method for computing the probability that a specific drug is effective or causes side effect in a subject, which comprises the steps of:

(a) computing diplotype distribution for the subject without family data using the method according to any one of [1] to [5]; and

(b) computing the probability that the specific drug is effective or causes side effect in the subject from the diplotype distribution computed in step (a) based on previously estimated or determined probability that the specific drug is effective or causes side effect in an individual having a specific diplotype configuration;

[8] a method for computing the probability that a subj ect develops or is affected with a disease, which comprises the steps of:

(a) computing diplotype distribution for the subject without family data using the method according to any one of [1] to [5]; and
(b) computing the probability that the subject develops or is affected with the disease from the diplotype distribution computed in step (a) based on previously estimated or determined probability that an individual having a specific diplotype develops or is affected with the disease;

[9] a program to compute diplotype distribution for each subject in a population from genotypic information of each subject, which comprises code means which when executed on a computer perform the step of the method according to [1];
[10] the program of [9], wherein the haplotype frequencies are computed in the population from genotypic information of each subject in the population just prior the step of computing the diplotype distribution;
[11] a program to compute the probability of a subject to exhibit a specific phenotype, which comprises code means which when executed on a computer perform the steps (a) and (b) of the method according to [6];
[12] a program to compute the probability that a specific drug is effective or causes side effect in a subject, which comprises code means which when executed on a computer perform the steps (a) and (b) of the method according to [7];
[13] a program to compute the probability that a subject develops or is affected with a disease, which comprises code means which when executed on a computer perform the steps (a) and (b) of the method according to [8];
[14] a computer-readable recording medium wherein the program according to any one of [9] to [13] has been stored;
[15] a device to compute diplotype distribution for each subject in a population without family data, which consists of:

(a) an input means for entering genotypic information of each subject in the population;
(b) a means for calculating haplotype frequencies from genotypic information of each subject in the population;
(c) a means for calculating diplotype distribution for each subject based on the haplotype frequencies in the population and the genotypic information of each subject; and
(d) a display means to display the calculated diplotype distribution;

[16] the device of [15], wherein step (b) is replaced with:

(b') an input means to enter previously estimated or determined haplotype frequencies of a population;

[17] a method for selecting a medical treatment appropriate for a subject, which comprises the steps of:

(a) computing the probability that a specific drug is effective or causes side effect in the subject by the method according to [7]; and
(b) determining whether to administer the drug, or the dose of the drug based on the probability computed in step (a); and

[18] a method for selecting a medical treatment appropriate for a subject, which comprises the steps of:

(a) computing the probability that the subject develops or is affected with a specific disease by the method according to [8]; and
(b) determining whether to perform treatment or prevention against the disease, or selecting a method for the treatment or prevention based on the probability computed in step (a).

The present invention provides a method of inferring diplotype configuration of each subject in a population based on the genotypic information of the subject without family data, i.e., a method for computing diplotype distribution for each subject. In the present invention, first, haplotype frequencies in a population are computed from genotypic information of each subject in the population (step (a)).

The term "genotype" normally refers to a combination of two alleles at a gene locus of an individual. For example, when a polymorphic site exists at a gene locus, a "genotype" indicates the characteristic of the two alleles of an individual corresponding to the polymorphic site. The phrase "genotypic information", as used herein, refers to the information on such genotypes. The genotypic information of the present invention may include information on a number of genotypes.

The characteristics of a gene serving as the genotypic information of the present invention include single nucleotide polymorphisms, microsatellites and mutations such as insertion/deletion. The genotypic information of the present invention is not limited to a single type of the above-mentioned polymorphisms or genetic mutations; it may contain information on multiple types of polymorphisms or genetic mutations.

When the genotypic information of the present invention includes information on a number of gene loci, the gene loci are preferably linked together. Thus, in the present invention, the genotypic information is preferably obtained on gene loci known to be linked together, but not restricted to such genotypic information, since in some cases it is not clear whether there is such a linkage or not.

As used herein, the term "haplotype" refers to a combination of alleles at multiple linked sites such as polymorphic sites. Generally, in any gene region, multiple polymorphic sites such as single base substitutions (SNP: single nucleotide polymorphism), microsatellites and insertion/deletion, exist proximal to each other and are linked together to form haplotypes. Every individual carries two haplotypes for each genetic region on the autosomes, one derived from the mother and the other from the father. This combination of two haplotypes of an individual is referred to as "diplotype configuration".

As used herein, the term "population" refers to a group of individuals providing genotypic information. To increase the reliability of diplotype configuration inferred according to the present invention, preferable population is composed of as many individuals as possible. For example, when the number of subjects in the population is 25 (the number of haplotypes = 50) or more and no rare haplotype is contained in the population, a diplotype configuration of an individual can be inferred with relatively high accuracy by the present method. Thus, the number of individuals in the population of the present invention is preferably, but not limited to, 25 or greater.

In addition, the population is preferably homogeneous (similar genetic background). For example, when a subject's diplotype configuration is to be determined for a Japanese, the "population" to obtain genotypic information is preferably a Japanese population. The homogeneity of a population can be determined by assessing whether respective gene loci meet the condition of the Hardy-Weinberg equilibrium.

A haplotype rarely changes during alteration of generations when contained in a narrow genetic region, because the haplotype changes due to crossover in a genetic region and the probability of crossover within a narrow genetic region is extremely low (the frequency is once within 100,000 kb in one generation). Therefore, the frequencies of respective haplotypes are often fixed in a particular population, such as Japanese. This is referred to as "haplotype frequency". In most cases, the haplotype frequency varies from population to population.

Any known method can be used for computing the haplotype frequency in the present step. Examples of preferred methods for computing the haplotype frequency include the maximum-likelihood method based on the EM algorithm (Excoffier, L. & Slatkin, M., 1995; Hawley, M. E. & Kidd, K. K., 1995; Long, J. C., et al., 1995; Fallin, D. & Schork, N. J. , 2000) ; analytical solutions (Elandt-Johnson, R.C., 1971; Hill, W.G, 1974; Yasuda, N., 1978; Imanishi, T., et al., 1991), and sequential haplotype-inferring algorithm (Clark, A. G., 1990).

When the EM algorithm is utilized, a haplotype frequency can be computed from genotypic information, as follows: Where n denotes the number of loci and $a_i$ the number of alleles at the i-th locus, total number of possible haplotypes at n loci N is given by $N = \Pi_{i=1}^{n} a_i$ . Suppose g represents the number of individuals without any familial data for whom the data on genotypes at n linked loci could be obtained. A maximum of $2^{n-1}$ types of diplotype configurations may exist for the genotypic data obtained for each individual. Where $f_i$ (i= 1, 2, ..., N) denotes the frequency of the i-th haplotype within the population, then $f_i \geqq 0$ (i= 1, 2, ..., N), and $\sum_{i=1}^{N} f_i = 1$ . When the population meets the condition of the Hardy-Weinberg equilibrium, the likelihood for a diplotype configuration of an individual can be computed by giving genotype data of the individual.

The procedure of EM algorithm to compute the maximum-likelihood estimate of $f_i$, $\hat{f}_i$ is as follows:

(1) A storage area is configured to store possible haplotypes in a population and suppose the total number of possible haplotypes is T. T=0 at the beginning.

(2) All possible diplotype configurations (combination of two haplotypes; a combination of two identical haplotypes may form one diplotype configuration) are listed for each individual by taking the given genotypes at every locus into consideration.

(3) As a result, the possible haplotype for the individual is determined. If this haplotype does not exist in the storage area, which stores the possible haplotypes in the population, the haplotype is added as a new possible haplotype and the haplotypes in the population are re-numbered. Then, T is appropriately increased.

(4) Next, for this individual, the previously listed possible diplotype configurations are represented by combinations of numbers of the possible haplotypes in the population (a diplotype configuration is a combination of two haplotypes), and the possible diplotype configurations for each individual are stored in the storage area.

(5) Following the listing of the possible diplotype configurations for each individual and possible haplotypes in the population, random positive real numbers are given as variables to $f_i$ (i= 1, 2, ..., T). Herein, $\sum_{i=1}^{T} f_i = 1$, where as described above, T denotes the number of all possible haplotypes in the population.

(6) All possible diplotype configurations (given as combinations of two numbered possible haplotypes in the population, as described above) listed for each individual in (4) consist of two haplotypes. The prior probability for each haplotype in an individual is taken as $f_i$ corresponding to the haplotype. Suppose a specific possible diplotype configuration of the individual to consist of i-th and j-th haplotypes and that the Hardy-Weinberg equilibrium can be applied, the likelihood for a diplotype configuration of the individual is represented by, when $i \neq j$, $2f_if_j$, and when $I=j$, $f_i^2$. The posterior probability for each possible diplotype configuration is computed from the likelihood of all possible diplotype configurations for the individual according to the Bayes' theorem.

(7) The posterior probability of each possible diplotype configuration for each individual is computed from the likelihood of each of the possible diplotype configurations according to Bayes' theorem.

(8) The posterior probabilities of possible diplotype and the joint likelihood are computed for each individual.

(9) The likelihood for all diplotype configurations of each individual are computed by multiplying the joint likelihood of each individual for all the individuals.

(10) The expected values for the numbers of respective haplotypes in the population are computed from the posterior probabilities of possible diplotype configurations for each individual in consideration of the numbers of the haplotypes forming each diplotype configuration.

(11) Thus computed expected values of the number of each haplotype are respectively divided by 2g to calculate the expected value for the frequency of each haplotype in the population.

(12) The data of haplotypes giving extremely low frequencies (for example, $<10^{-4}$) among the computed haplotype frequencies are removed from the storage area where the possible haplotypes in the population have been stored. Then, the list is rearranged and new number T for the possible haplotypes in the population is configured.

(13) The expected value for the frequency of each haplotype in the population that was computed in (11) is assigned into $f_i$ (i= 1, 2, ..., T).

(14) The value determined in (13) is assumed as the frequency of a haplotype in the population, and then the process is resumed from step (6). The steps (6) to (13) as a cycle is repeated. Finally, when all of the likelihood for the diplotype configurations of each individual calculated in a cycle is extremely low (e.g., only an increase of $10^{-5}$) compared to that calculated in the previous cycle, the iteration is terminated and the likelihood is assessed to have converged on an approximate value.

(15) The computation described above gives the maximum-likelihood estimates of frequencies of the haplotypes in the population. Namely, the expected value for the frequency of each haplotype in the population computed in step (11) of the final cycle is the maximum-likelihood estimate.

According to the present invention, the diplotype distribution for each individual is then computed based on the haplotype frequencies computed in step (a) and the genotypic information for each individual (step (b)).

As used herein, the phrase "diplotype distribution" refers to a distribution of probabilities (posterior probabilities) for the entire possible diplotype configurations for each individual, i.e., the probability distribution of diplotype configurations for each individual. The diplotype distribution is obtained by predicting, in terms of probabilities, the diplotype configuration for each individual based on the genotypic information of the individual. Therefore, as used herein, the phrase "computing diplotype distribution for an individual" specifically means inferring the diplotype configuration of the individual.

Any known method can be used to compute the diplotype distribution in this step. Such methods for computing the diplotype distribution include the Bayes' theorem and calculation of the posterior probability. Specifically, examples of methods for computing the diplotype distribution include those described below. Namely, (1) taking each of the haplotype frequencies computed in step (a) as a prior probability; (2) performing the computation of steps (6) and (7); and (3) calculating the posterior probabilities for possible diplotype configurations for each individual as the diplotype distribution for each individual. For example, suppose k-th possible diplotype for an individual as $D_k$, and the combination of haplotypes constituting the diplotype as $H_i/H_j$. Herein, $H_i$ and $H_j$ denote i-th and j-th haplotypes in a population, respectively. When the maximum-likelihood estimates of the frequencies of the i-th and j-th haplotypes in the population are respectively represented by $\hat{f}_i, \hat{f}_j$, the likelihood $L_k$ for this diplotype configuration $D_k$ of this individual can be computed as follows:

$$L_k = \begin{cases} \hat{f}_i^{\,2} & \text{if } i = j \\ 2\hat{f}_i\hat{f}_j & \text{if } i \neq j \end{cases}.$$

Suppose the total number of possible diplotype configurations for this individual as M. The posterior probability $P_m$ of m-th diplotype configuration $D_m$ of the individual is computed by following formula:

$$P_m = L_m / \sum_{i=1}^{M} L_i \quad .$$

The posterior probability $P_m$ is computed for m=1, 2, ..., M, as the diplotype distribution of this individual.

The present inventors found that in most cases the diplotype distribution generally concentrates in a single diplotype configuration. Thus, when the diplotype distribution computed in the above-described step (b) is concentrated in a single diplotype configuration, the individual is predicted to have this concentrated diplotype configuration. Alternatively, when the diplotype distribution is not concentrated in a single diplotype configuration, the probabilities of multiple diplotype configurations for the individual are computed as the diplotype distribution. For example, in an individual, the probability of having diplotype configuration A is computed to be X, and the probability of having diplotype configuration B is computed to be Y.

Alternatively, in the present invention, instead of the haplotype frequencies computed from genotypic information for each individual in a population via the method described above, previously estimated or determined haplotype frequencies can also be used to compute the diplotype distribution. Thus, the present invention provides a method for computing a diplotype distribution for an individual without family data, which comprises the step of computing the diplotype distribution for the individual based on previously estimated or determined haplotype frequency of a population and genotypic information of the individual.

The "previously estimated or determined haplotype frequency of a population" is preferably the haplotype frequency of a population that includes the individual for whom the diplotype configuration is inferred (computation of diplotype distribution). However, a haplotype frequency computed without the genotypic information for the individual may also be used. This method of the present invention has the advantage that a diplotype configuration of an unknown individual can be inferred based on only the genotypic information of the individual by the use of previously estimated or determined haplotype frequencies of a population.

The "previously estimated or determined haplotype frequency of a population" may be estimated or determined according to the above-described method of the present invention, for example, those derived from genotypic information of an individual via the maximum-likelihood method based on the EM algorithm.

The diplotype distribution computed according to the method of the present invention is widely applicable due to its high reliability. In one embodiment, the present invention provides a method for computing the probability that a subject exhibits a specific phenotype based on (1) the diplotype distribution for an individual computed according to the method of the present invention, and (2) previously estimated or determined probability (penetrance) that an individual with a specific diplotype configuration has the specific phenotype.

As used herein, the term "phenotype" refers to a characteristic that is intrinsic to an individual and different among individuals and which is associated with diplotype configuration of a gene. These include such characteristics as an individual's susceptibility to a disease, response to a drug, or side effects thereto. The present method is performed based on the comparison between a diplotype distribution for a subject (computed by the method of the present invention) and previously estimated or determined probability that an individual with a specific diplotype configuration exhibits a specific phenotype. For example, suppose there is previously estimated or determined information that the probability that an individual with diplotype A exhibits phenotype B is X. Then, when a subject is inferred to have a diplotype configuration A (with 100% probability) (when the diplotype distribution is concentrated alone in the diplotype configuration A), the probability that the subject exhibits the phenotype B is computed to be X.

When a diplotype distribution of an individual determined by the method of the present invention is not concentrated in a single diplotype configuration, the probability that the individual exhibits a specific phenotype can be computed, for example, as follows. Suppose a subject to have a total of M types of diplotype configurations $D_k$ (k= 1, 2, ..., M) in a diplotype distribution, and the posterior probability for $D_k$ is $P_k$ ($\Sigma P_k = 1$). When the probability (i.e., penetrance) that $D_k$ exhibits phenotype A has been estimated or determined to be $A_k$, the probability $P_{af}$ that the subject develops the phenotype A can be computed according to following formula:

$$P_{af} = \sum_{k=1}^{M} P_k A_k \quad .$$

As an alternative embodiment of the use of diplotype distribution computed by the method of the present invention, the present invention provides a method for computing the probability that a specific drug is effective or causes side effect in a subject. The computing is conducted based on (1) the diplotype distribution of the subject computed according to the present method, and (2) previously estimated or determined probability that the drug is effective or causes side effects in an individual having a specific diplotype configuration.

The computing of the above-mentioned probability in the present method is performed based on the comparison between (1) the diplotype distribution for a subject computed according to the method of the present invention, and (2) previously estimated or determined probability that a drug is effective or causes side effects in an individual having a specific diplotype configuration. For example, suppose X as the probability that drug B is effective in an individual with a diplotype configuration A, and Y the probability that the drug causes side effect in the same individual; then, when the diplotype configuration of a subject is inferred to be A (when diplotype distribution is concentrated in only the diplotype configuration A), the probability that the drug B is effective in the subject is computed to be X and the probability that the drug causes side effect in the subject is computed to be Y.

When a diplotype distribution of an individual determined by the method of the present invention is not concentrated in a single diplotype configuration, the probability that a specific drug is effective or causes side effect in a subject can be computed by the following procedure. Suppose the subject to have a total of M types of diplotype configurations $D_k$ (k= 1, 2, ..., M) in a diplotype distribution, and $P_k$ as the posterior probability for $D_k$ ($\Sigma P_k = 1$). When the probability that a specific drug is effective or causes side effect in $D_k$ has been estimated or determined to be $E_k$, the probability $P_{ef}$ that the specific drug is effective or causes side effect in the subject is computed according to the following formula:

$$P_{ef} = \sum_{k=1}^{M} P_k E_k \ .$$

As an alternative embodiment of the use of diplotype distribution computed via the method of the present invention, the present invention also provides a method for computing the probability that a subject develops or is affected with a specific disease, based on the diplotype configuration of the subject computed using the method of the present invention and previously estimated or determined probability that an individual with a specific diplotype configuration develops or is affected with the specific disease.

This method is performed based on the comparison between a diplotype distribution for the subject computed via the method of the present invention and previously estimated or determined probability that an individual with a specific diplotype configuration develops or is affected with a specific disease. For example, suppose X as the probability that an individual having diplotype A develops disease B, and Y as that that the same individual is affected with the disease; if the diplotype configuration inferred for a subject is A (when the diplotype distribution is concentrated in only the diplotype configuration A), the probability that the subject develops disease B is computed to be X and the probability that the subject is affected with the disease is computed to be Y.

When a diplotype distribution of an individual computed by the present method is not concentrated in a single diplotype configuration, the probability that a subject develops or is affected with a specific disease can be computed by the following procedure. Suppose an individual to have a total of M types of diplotype configurations $D_k$ (k= 1, 2, ..., M) in a diplotype distribution, and $P_k$ as the posterior probability for $D_k$ ($\Sigma P_k = 1$). When the probability that $D_k$ develops or is affected with a specific disease has been estimated or determined to be $B_k$, the probability $P_{bf}$ that a subject develops or is affected with the specific disease can be computed by the following formula:

$$P_{bf} = \sum_{k=1}^{M} P_k B_k \ .$$

The present invention also provides computer programs that can be used to perform the methods of the present invention described above. Below appear the computer programs (1)-(5) of the present invention:

(1) A computer program to compute, a haplotype frequency in a population from genotypic information of each individual in the population, and then a diplotype distribution for each individual in the population from the computed haplotype frequency and the genotypic information of each individual in relation to the present method to compute the diplotype distribution for an individual in the population without family data.

(2) A computer program to compute, a diplotype distribution for an individual in a population from previously estimated or determined haplotype frequency of the population and genotypic information of the individual in relation to the present method to compute the diplotype distribution for a subject without family data.

(3) A computer program to compute, a diplotype distribution of a subject via the method of the present invention, and the probability that a subject exhibits a specific phenotype from the computed diplotype distribution based on previously estimated or determined probability that an individual with a specific diplotype configuration exhibits the specific phenotype in relation to the present method to compute the probability that a subject exhibits a specific phenotype.

(4) A computer program to compute, a diplotype distribution of a subject via the method of the present invention, and from the computed diplotype distribution, the probability that a specific drug shows effect or side effect in a subject based on previously estimated or determined probability that the specific drug shows effect or side effect in an individual having a specific diplotype configuration in relation to the present method to compute the probability that a specific drug shows effect or side effect in a subject.

(5) A computer program to compute, a diplotype distribution for a subject via the method of the present invention, and the probability that a subject develops or is affected with a specific disease from the computed diplotype distribution based on previously estimated or determined probability that an individual having a specific diplotype configuration develops or is affected with the specific disease in relation to the present method to compute the probability that a subject develops or is affected with a disease.

Furthermore, the present invention provides computer-readable recording media stored with a computer program of the present invention descried above. The recording media of the present invention are readable by any general-purpose computer and record a computer program of the present invention. The recording media of the present invention include both transportable and stationary media, for example, CD-ROM, flexible disk (FD), DVD, hard disk, semi-conductor memory, etc.

In addition, the program of the present invention can be commercialized as a program stored in the above-mentioned transportable recording medium. Alternatively, the program can be stored in a recording device of a computer on a network and then transferred to other computers via the network. The computer program to achieve the process of the present invention can be distributed to users as a computer-readable medium of various formats. The present invention is applicable for actual distribution to any media irrespective of the type of distribution medium.

The present invention also provides a device to compute diplotype distribution for each individual in a population without family data. Such a device comprises an input means to enter genotypic information of each individual in the population, a means to calculate haplotype frequencies in the population based on the genotypic information, another means to calculate the diplotype distribution of an individual from the haplotype frequencies in the population and the genotypic information of each individual, and means to display the calculated diplotype distribution.

In addition, the present invention provides a device to compute diplotype distribution of a subject without family data. The device comprises of an input means to enter genotypic information of the subject and previously estimated or determined haplotype frequencies in a population, a means to calculate the diplotype distribution for the subject from the genotypic information of the subject and the previously determined haplotype frequencies in the population, and a display means to display the calculated diplotype distribution.

According to a preferred embodiment of the above-described device of the present invention, the device is a computer wherein the programs of the present invention have been stored in an auxiliary storage device, such as a hard disk. The programs comprise a program module to compute haplotype frequencies, a program module to compute diplotype distribution and a program module to control the program modules. However, the program module to compute haplotype frequencies can be omitted, when previously estimated or determined haplotype frequencies are used as the input data.

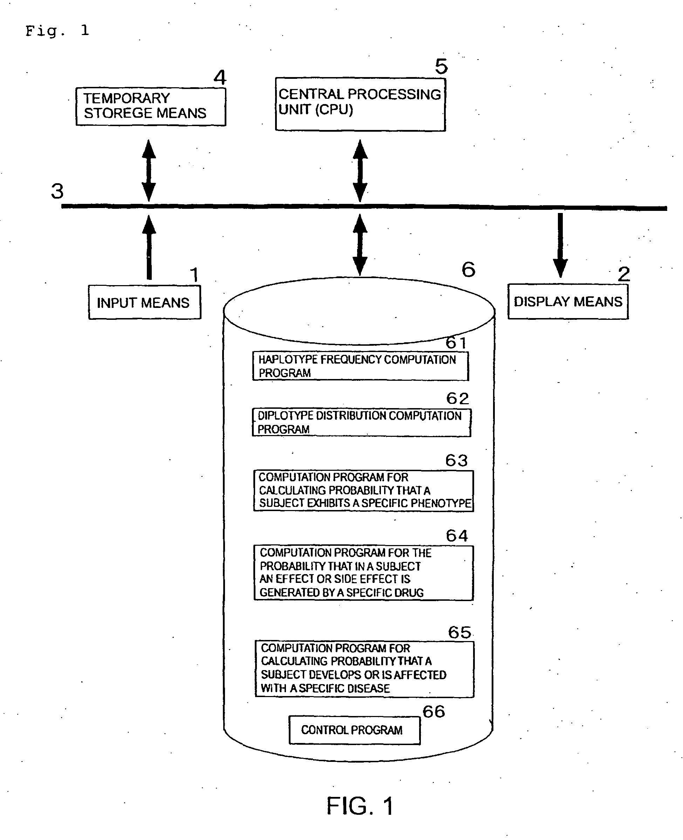

Fig. 1 shows an example illustrating the systemic structure of the device of the present invention. Input means 1 and display means 2 have been connected to bus 3. Temporary storage means 4 temporarily stores input data, computed numeric data and other data. Central processing unit (CPU) 5 executes a variety of operations at the command of the program of the present invention. Main memory 6 stores various programs required to execute processing of the present invention. These programs can be roughly classified into haplotype frequency computation program 61; diplotype distribution computation program 62; computation program 63 to calculate the probability of an individual to exhibit a specific phenotype; computation program 64 to compute the probability of an individual to show effect or side effect against a specific drug; calculation program 65 to compute the probability of a subject to develop or to be affected with a specific disease; and control program 66 to control these programs. These programs 61 to 66 can be integrated into a single program. The above-described program 61 for computing haplotype frequencies can be omitted, when previously estimated or determined haplotype frequencies are entered through the input means. Alternatively, the above-described programs 63 to 65 can be omitted, when the purpose is to compute diplotype frequencies for an individual.

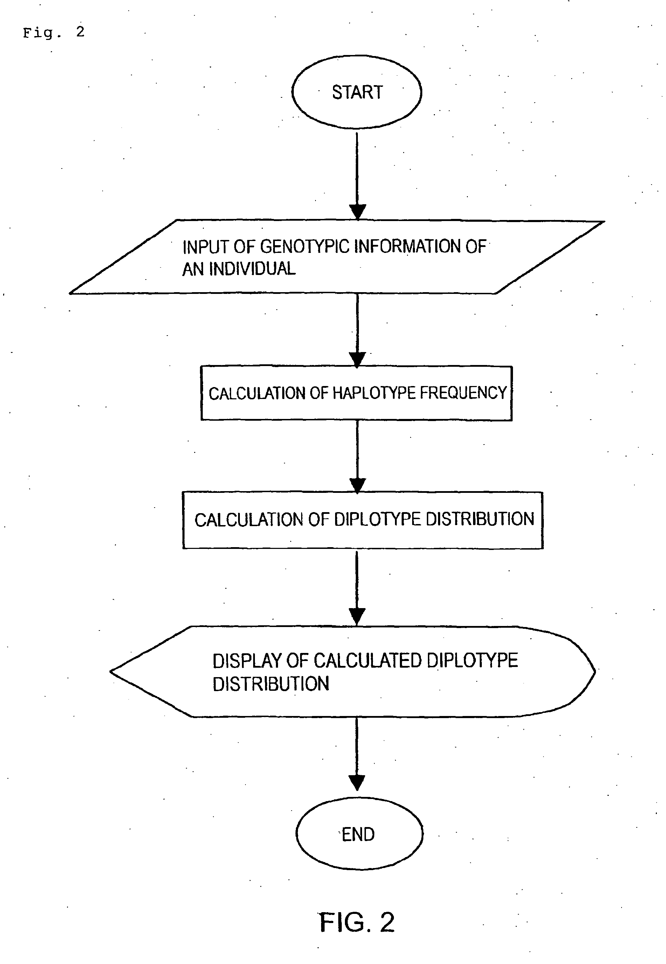

Fig. 2 shows an exemplary flowchart of the process executed by the device. First, genotypic information(s) of individual(s) (hereinafter, in some cases, simply refers to as "data") is entered via the input means. When previously estimated or determined haplotype frequencies are used, the haplotype frequencies are also entered. In addition to directly entering the genotypic information and, if required, the haplotype frequencies into a computer via an input means, such as keyboard, the information can be entered into the computer using transportable recording media or stationary media (e.g., hard disk) carrying the genotypic information and/or haplotype frequencies. Alternatively, using a receiving means such as modem the information can be entered into a computer from a communication network, such as internet databank. Furthermore, appropriate data may be pre-stored in a stationary medium (e.g., hard disk) to appropriately load the data from the stationary medium. The entered data can be stored in the main memory or in a temporary storage means of the computer.

Then, haplotype frequency in a population is computed from the entered genotypic information of the individual by the above-described method. Generally, this process to calculate the haplotype frequency is executed in a means, such as a central processing unit (CPU). The haplotype frequency computation program within the main memory instructs to read out genotypic information, and based on the information, the means calculates the haplotype frequency. The computed result (haplotype frequency) is stored in a storage means or in a temporary storage means of the computer, and used for the computation of diplotype distribution. However, this step can be omitted when previously estimated or determined haplotype frequencies are entered.

Next, the diplotype distribution is computed from the genotypic information and haplotype frequencies according to the above-mentioned method. This process is executed in a means, such as a central processing unit (CPU) following the instructions of the diplotype distribution computation program within the main memory. Namely, based on the genotypic information entered as described above and the haplotype frequencies entered or stored in the storage means, the diplotype distribution is computed in the means.

Then, the calculated diplotype distribution is displayed by a display means. This display means includes printers and such in addition to display monitors.

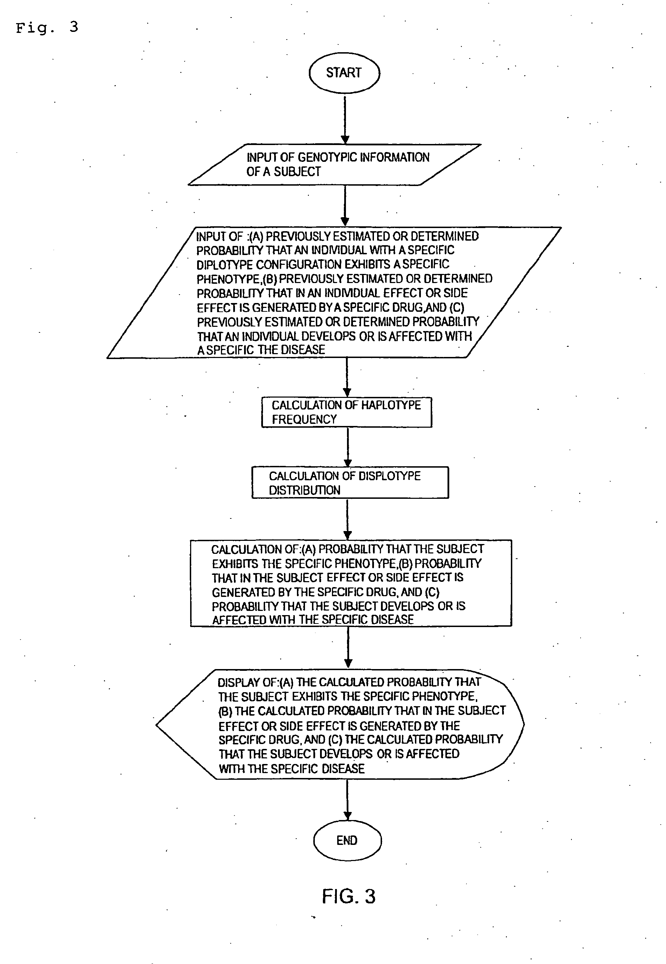

Fig. 3 shows a flowchart of the process for computing with the device of the present invention, the probability that an individual exhibits a specific phenotype, the probability that an individual shows effect or side effect against a specific drug, and the probability that an individual develops or is affected by a specific disease. In this process, in addition to the steps indicated for the process described above in Fig. 2, previously estimated or determined probability that an individual having a specific diplotype configuration (a) exhibits a specific phenotype, (b) shows effect or side effect against a specific drug, or (c) develops or is affected with a specific disease, is entered from an input means. Then, upon receiving instructions from the program within the main memory, based on the entered data and the diplotype distribution that was computed according to the above-described steps of the procedure, a means, such as a central processing unit (CPU), calculates the probability that the subject: (a) exhibits the specific phenotype, (b) shows effect or side effect against the specific drug, or (c) develops or is affected with the specific disease. Finally, the computed probability is displayed via a display means.

The present invention is related to a database stored in a machine-readable medium composed of data of: (1) computed haplotype frequencies in a population, and/or (2) computed diplotype distribution, in relation to the present method for computing diplotype distribution.

The database described herein may comprise a so-called "database schema" in addition to the above-described data. The "database schema" defines the format and relation of accumulated data in a database. It is generally described in database language.

The database described herein may be distributed or marketed stored in a storage medium, such as CD-ROM. Alternatively, users may access online the place where the database has been stored and maintained to inquire for the database. The database may be made available on the internet to enable Web browser-based online search. For example, a diplotype distribution of an individual may be computed by the present method based on the data of haplotype frequencies that may be obtained through database queries.

The present invention further provides a method for selecting a medical treatment adapted for an individual. In one embodiment of the method, a determination whether a drug should be administered or determination of the dose of the drug is made based on the probability that the individual shows effect or side effect against the specific drug, that is calculated using the above-described method of the present invention.

For example, when the probability that the specific drug is effective, computed according to the above-described method, is high, the drug is administered to the subject. Conversely, when the computed probability is low, the drug is not administered. Alternatively, when the computed probability that the drug causes side effect is high, the administration of the drug is avoided or the dose is decreased. When the computed probability to cause side effect is low, the drug is administered at a therapeutically effective dose.

In an alternative embodiment of the present method for selecting a medical treatment adopted for an individual, a determination whether a treatment or prevention against a disease should be practiced or a determination to select a method for treatment or prevention is made based on the probability that the individual develops or is affected with the disease that has been computed using the above-described method of the present invention.

For example, when the probability that a subject develops a disease, computed according to the above-described method, is high, the subject is subjected to a preventive treatment against the disease. When the probability to develop the disease is low, no treatment is made. Alternatively, when the probability that the subject is affected with the disease is high, detailed examination on symptoms related to the disease should be conducted by physicians to appropriately give effective treatments. When the probability of being affected with the disease is low, no close examination for the disease is needed.

Hitherto, most medical treatments were so-called ready-made medicine that are uniformly performed almost without considering the differences among individuals. The above-described method of the present invention enables very personal selection of therapeutic drugs, prescription, such as dose, and therapeutic method adapted for different constitution due to the genetic variations among individuals. Thus, according to the present invention, it is expected that medical treatments (tailor-made medicine) that are more effective or have decreased side effect can be performed on individual patients.

Furthermore, according to the present invention, a diplotype configuration or diplotype distribution for an individual can be obtained based on genotypic information. Based on these data, the probability that a specific drug is effective (Re) or caused side effect (Rs) in a subject who has the diplotype configuration can be computed. By showing the rates of these two probabilities to a patient, it is expected that the patient can take the lead to determine whether to take the drug with reference to physician's advice.

Brief Description of the Drawings

Fig. 1 depicts an illustration showing the systemic structure of the device of the present invention.
Fig. 2 shows an exemplary flowchart of the process executed by the device of the present invention.
Fig. 3 shows an exemplary flowchart of the process executed by the device of the present invention.
Fig. 4 shows a schematic illustration of the genomic structure of the smoothelin gene.
Fig. 5 shows a schematic illustration of the genomic structure of the serum amyloid A (SAA) gene.

(Explanation of symbols)

1. Input means
2. Display means
3. Bus
4. Temporary storage means
5. Central processing unit (CPU)
6. Main memory

Best Mode for Carrying out the Invention

The present invention is illustrated in detail below with reference to Examples, but is not to be construed as being limited thereto.

Example 1: Determination of diplotype configuration for smtn gene by *in-vitro* experiments

Two discrete regions, smlr-1 and smlr-2, were selected among various polymorphic regions of genes. Each of the regions contains two or more polymorphic gene loci. The two regions were analyzed by following procedure using PCR.

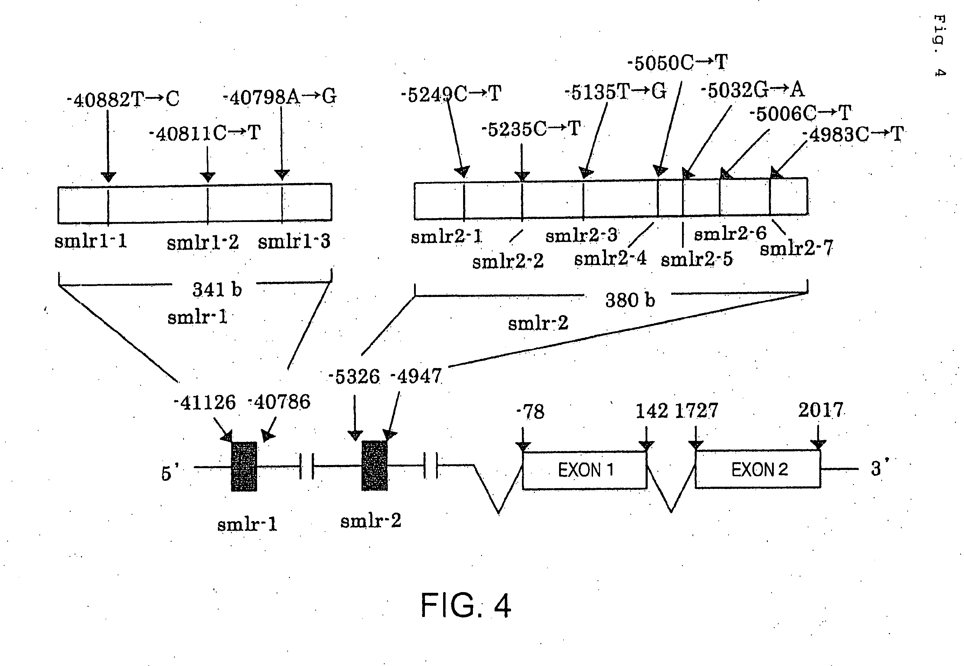

Fig. 4 shows a physical map of smtn. PCR amplification was carried out in a 20 $\mu$l solution containing standard buffer (10 mM Tris-HCl, pH 8.3, 50 mM KCl, 1.5 mM MgCl$_2$ and 0.001% gelatin), dNTPs (200 $\mu$M each), primers (0.5 $\mu$M each), AmpliTaq Gold polymerase (0.5 U; ABI) and genomic DNA (5 ng). The profile of thermal cycling consisted of: 36 cycles of first denaturation at 94˚C for 9 min or denaturation at 95˚C for 30 sec, primer annealing at 54˚C for 1 min for smlr-1 or at 56˚C for 1 min for smlr-2, and extension at 72˚C for 1 min. After the 36th cycle, the final extension was carried out at 72˚C for 15 min. The sense and antisense primers for smlr-1 were: 5'-TAGGAACCTAGTAGGTCCTTA-3' (SEQ ID NO: 1) and 5'-AAGGAAATAGTTGTAGCCACTA-3' (SEQ ID NO: 2), respectively. On the other hand, the sense and antisense primers for smlr-2 were: 5'-ACTGCCTCCGTGCCATTTG-3' (SEQ ID NO: 3) and 5'-CAGATCCAGTAAGCCTCAC-3' (SEQ ID NO: 4), respectively. The smlr-1 region covers 341 bp from -41126 to -40786; and the smlr-2 region covers 380 bp from -5326 to -4947. The types and sites of SNPs are listed in Fig. 4. Genotypes were determined by DHPLC and direct sequencing of the amplified product. DNAs were purified using PCR Product Pre-Sequencing kit (USB), and then direct sequencing was performed using ABI Prism Big Dye Terminator Cycle Sequencing Ready Reaction Kit and model 3700 DNA sequencer. The same amplified sequences were also applied to DHPLC. DHPLC was carried out to physically determine diplotype configurations using WAVE DNA fragment analysis system (Transgenomics) according to manufacturer's instructions. Since multiple polymorphic gene loci on the same chromosome are amplified as a single molecule by PCR, the haplotype can be determined by DHPLC.
The haplotype was also determined by fluorescent SSCP using the same sense and antisense primers described above that had been labeled with FAM and NED, respectively. Samples for SSCP were prepared by PCR similarly to the procedure described above. The amplified product was combined with an equal volume of sequencing buffer, heat-denatured and then supplied to ABI Prism 377 DNA sequencer for analysis with the Gene Scan program. 4.5% polyacrylamide gel (acrylamide: bisacrylamide = 99:1) containing 1x TBE buffer was used as analytical gel. Analysis was also carried out using gels containing 10% glycerol to improve the accuracy of SSCP. All analyses were performed at 20 ˚C.

The diplotype configuration for each subject was decided from the two haplotypes in the sample that were determined as described above. Thus determined haplotype frequencies were completely different from those computed on the assumption that all three ethnic groups are free from linkage disequilibrium. Table 1 shows a comparison of numbers and frequencies of the haplotypes for the two regions of the smoothelin gene, smlr-1 and smlr-2, between those determined in the *in-vitro* experiments and those computed according to the EM algorithm. Table 2 shows the numbers of persons having each diplotype configuration determined physically (left) and computed according to the EM algorithm (right) for the two regions of the smoothelin gene, smlr-1 (panel A) and smlr-2 (panel B).

Table 1

| region | ethenic group[a] | haplotype | number of haplotype | haplotype frequency | | |
|---|---|---|---|---|---|---|
| | | | | haplotype frequency determined in the in-vitro experiments | haplotype frequency calculated according to the EM algorithm | haplotype frequency calculated on the assumption that SNPs at different sites are independent of one another [c] |
| smlr-1 | J | CA[d] | 34 | 0.531 | 0.531 | 0.571 |
| | | CG | 7 | 0.109 | 0.109 | 0.070 |
| | | TA | 23 | 0.359 | 0.359 | 0.320 |
| | | TG | 0 | 0 | 0 | 0.039 |
| | EA | CA[d] | 18 | 0.281 | 0.281 | 0.395 |
| | | CG | 15 | 0.234 | 0.234 | 0.121 |
| | | TA | 31 | 0.484 | 0.484 | 0.371 |
| | | TG | 0 | 0 | 0 | 0.114 |
| | AA | TCA[e] | 23 | 0.359 | 0.359 | 0.440 |
| | | TCG | 13 | 0.203 | 0.203 | 0.135 |
| | | TTA | 26 | 0.406 | 0.406 | 0.301 |
| | | CCG | 2 | 0.031 | 0.031 | 0.004 |
| | | others | 0 | 0 | 0 | 0.120 |

(continued)

| region | ethenic group[a] | haplotype | number of haplotype | haplotype frequency | | |
|---|---|---|---|---|---|---|
| | | | | haplotype frequency determined in the in-vitro experiments | haplotype frequency calculated according to the EM algorithm | haplotype frequency calculated on the assumption that SNPs at different sites are independent of one another [c] |
| smlr-2 | J | TCT[f] | 57 | 0.891 | 0.891 | 0.795 |
| | | TTC | 6 | 0.094 | 0.094 | 0.010 |
| | | GCC | 1 | 0.016 | 0.016 | 0.002 |
| | | others | 0 | 0 | 0 | 0.193 |
| | EA | TCT[f] | 53 | 0.828 | 0.828 | 0.691 |
| | | TTC | 8 | 0.125 | 0.125 | 0.020 |
| | | GCC | 3 | 0.047 | 0.047 | 0.007 |
| | | others | 0 | 0 | 0 | 0.282 |
| | AA[b] | CCTCGTC[g] | 33 | 0.532 | 0.548 | 0.264 |
| | | CCGCGCT | 14 | 0.226 | 0.210 | 0.023 |
| | | CCTTGCC | 9 | 0.145 | 0.145 | 0.032 |
| | | CCGCGCC | 3 | 0.048 | 0.048 | 0.078 |
| | | TCTCGTC | 1 | 0.016 | 0.016 | 0.004 |
| | | CCGCACT | 0 | 0 | 0.016 | 0.000 |
| | | CCGCGTC | 1 | 0.016 | 0.016 | 0.108 |
| | | CCTCATC | 1 | 0.016 | 0 | 0.004 |
| | | others | 0 | 0 | 0 | 0.487 |

[a]J: Japanese, EA: European-American, AA: African-American.

[b]Data from a subject was eliminated due to its ambiguity.

[c]Haplotype frequencies computed from SNP frequencies on the assumption that SNPs at different sites are independent of one another.

[d]Haplotype containing the SNP loci, smlr-1-2 and smlr-1-3, shown in Fig. 4.

[e]Haplotype containing the SNP loci, smlr-1-1, smlr-1-2 and smlr-1-3, shown in Fig. 4.

[f]Haplotype containing the SNP loci, smlr-2-3, smlr-2-4 and smlr-2-6, shown in Fig. 4.

[g]Haplotype containing the SNP loci, smlr-2-1, -2, -3, -4, -5, -6 and -7, shown in Fig. 4.

Table 2

(panel A)

|  | haplotype[a] | TTA | TCA | TCG | CCG |
|---|---|---|---|---|---|
| African-American | TTA | 6/6 | 7/7 | 6/6 | 1/1 |
|  | TCA | — | 5/5 | 5/5 | 1/1 |
|  | TCG | — | — | 1/1 | 0/0 |
|  | CCG | — | — | — | 0/0 |
| Japanese | haplotype[b] | CA | TA | CG |  |
|  | CA | 12/12 | 8/8 | 2/2 |  |
|  | TA | — | 6/6 | 3/3 |  |
|  | CG | — | — | 1/1 |  |
| European-American | haplotype[b] | TA | CA | CG |  |
|  | TA | 9/9 | 9/9 | 4/4 |  |
|  | CA | — | 2/2 | 5/5 |  |
|  | CG | — | — | 3/3 |  |

(panel B)

| | haplotype[d] | 0 | 1 | 2 | 3 | 4 | 5 | 6 | 7 |
|---|---|---|---|---|---|---|---|---|---|
| African-American | 0 | 11/11 | 7/7 | 3/3 | 0/0 | 1/1 | 0/0 | 0/1 | 0/0 |
| | 1 | — | 0/0 | 4/4 | 1/1 | 0/0 | 1/1 | 0/0 | 1/0 |
| | 2 | — | — | 1/1 | 0/0 | 0/0 | 0/0 | 0/0 | 0/0 |
| | 3 | — | — | — | 1/1 | 0/0 | 0/0 | 0/0 | 0/0 |
| | 4 | — | — | — | — | 0/0 | 0/0 | 0/0 | 0/0 |
| | 5 | — | — | — | — | — | 0/0 | 0/0 | 0/0 |
| | 6 | — | — | — | — | — | — | 0/0 | 0/0 |
| | 7 | — | — | — | — | — | — | — | 0/0 |

| | haplotype[e] | TCT | TTC | GCC |
|---|---|---|---|---|
| Japanese | TCT | 25/25 | 6/6 | 1/1 |
| | TTC | — | 0/0 | 0/0 |
| | GCC | — | — | 0/0 |
| European-American | haplotype[e] | TCT | TTC | GCC |
| | TCT | 23/23 | 5/5 | 2/2 |
| | TTC | — | 1/1 | 1/1 |
| | GCC | — | — | 0/0 |

[a]Haplotype containing the SNP loci, smlr-1-1, smlr-1-2 and smlr-1-3, shown in Fig. 4.

[b]Haplotype containing the SNP loci, smlr-1-2 and smlr-1-3, shown in Fig. 4.

[c]Data from a subject was eliminated due to its ambiguity.

[d]Haplotype containing the SNP loci, smlr-2-1, -2, -3, -4, -5, -6 and -7, shown in Fig. 4. The haplotypes were numbered as follows: 0, CCTCGTC; 1, CCGCGCT; 2, CCTTGCC; 3, CCGCGCC; 4, TCTCGTC; 5, CCGCGTC; 6, CCGCACT; 7, CCTCATC.

[e]Haplotype containing the SNP loci, smlr-2-3, smlr-2-4 and smlr-2-6, shown in Fig. 4.

Example 2: Computation of haplotype frequencies based on the EM algorithm and determination of diplotype distribution of smtn for each subject

The haplotype frequencies were calculated using the same data for smtn as in Example 1. The data of the three ethnic groups were analyzed separately. Data in the output file showed that the diplotype distributions of smlr-1 and smlr-2 regions for each subject were concentrated in a single diplotype configuration without exception. Furthermore, with respect to all the three ethnic groups except one African-American subject, this result agreed with that physically determined in *in-vitro* experiments. Table 2 shows the numbers of subjects having respective diplotype configurations. As shown in Table 2, in the three ethnic groups, no subject exhibited diplotype distribution to have two or more diplotype configurations. However, a diplotype configuration of one subject differed from the physically determined result (1.1%). As the conclusion, for both of the regions and all ethnic groups, the expected value of each of the haplotype frequencies was very close to the percentage of the haplotypes in the sample (Table 1).

Example 3: Computation for smtn using combined data sets

The data of two or more ethnic groups were combined to execute the program. The data of only two SNP loci (smlr-1-2 and smlr-1-3) were used for smlr-1 to obtain results from all the ethnic groups. Data of three SNP loci (smlr-2-3, -4 and -6) were used in the haplotype analysis for smlr-2, because those of the two other gene loci were only available from the African-American subjects.

In all cases, the diplotype distribution for each subject concentrated in a single diplotype configuration. Furthermore, these diplotype configurations agreed with those physically determined in *in-vitro* experiments. Not results for all the possible combinations (four possible combinations) of the three groups are shown but Table 3 depicts results of combinations of genotypic data for Japanese and European-Americans. As the conclusion, the computed haplotype frequencies agreed with those computed from data obtained by *in-vitro* experiments.

Table 3

| smlr-1 | haplotype[a] | TA | CA | CG | |
|---|---|---|---|---|---|
| | TA | 21/21 | 24/24 | 14/14 | |
| | CA | - | 19/19 | 13/13 | |
| | CG | - | - | 5/5 | |
| smlr-2 | haplotype[b] | TCT | TTC | GCC | GCT |
| | TCT | 60/60 | 14/14 | 11/11 | 0/0 |
| | TTC | - | 2/2 | 5/5 | 0/0 |
| | GCC | - | - | 3/3 | 1/1 |
| | GCT | - | - | - | 0/0 |

The data from the three groups (Japanese, European-Americans and African-Americans) were combined and applied to ldsupport software. None had diplotype distribution that concentrates in two or more diplotype configurations.
[a]Haplotype containing the SNP loci, smlr-1-2 and smlr-1-3, shown in Fig. 4.
[b]Haplotype containing the SNP loci, smlr-2-3, smlr-2-4 and smlr-2-6, shown in Fig. 4.

Example 4: Haplotype frequency and diplotype configuration of saa1 for each subject determined by *in-vitro* experiments

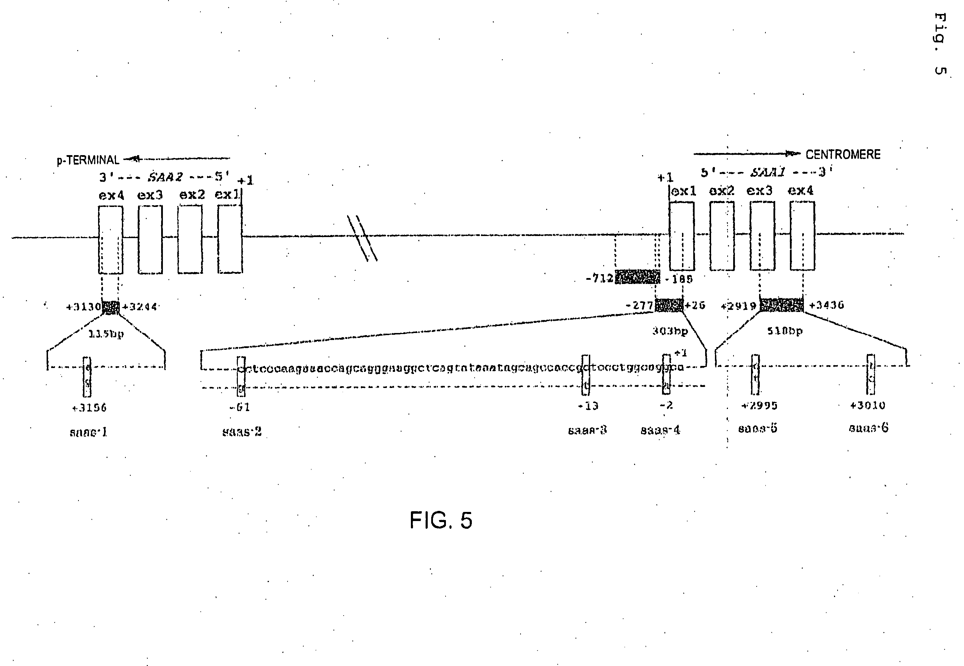

Two common SNP loci, saas-5 and saas-6, within the saa1 gene were selected because they are located within a short region that can be amplified by a single PCR (Fig. 5). After PCR, the diplotype configuration for each subject was determined through the identification of the two haplotypes by following method.

The genotypic data for the SAA gene were collected for all six SNP loci. Five of the SNP loci, saas-2, -3, -4, -5 and -6, are located within saa1, and the remaining SNP locus, saas-1, exists in the SAA2 gene (saa2) that is closely linked to saa1 (Fig. 5). Among them, two common SNP loci, saas-5 and saas-6, exist proximally and therefore can be analyzed simultaneously in a single DNA segment amplified by PCR (Baba, S., et al. (1995) A novel allelic variant of serum amyloid A, SAA1 gamma: genomic evidence. Evolution, frequency, and implication as a risk factor for reactive systemic AA-amyloidsis. Hum. Mol. Genet. 4, 1083-1087). Thus, a 518-bp segment starting from position 2919 of saa1, which contains the two SNP loci (at positions 2995 and 3010), was amplified by PCR according to the method described in literature (Baba, S., et al., 1995). Next, the nucleotide sequences of amplified segments were determined to identify the SNPs. The amplified sequences were digested with *Ban* I or *Bcl* I to confirm the presence of polymorphism (Yamada, T., et al. (1999) Serum amyloid A1 alleles and plasma concentrations of serum amyloid A. Amyloid 6, 199-204). The amplified sequences were subjected to previously reported technique for single strand conformational polymorphism (SSCP; Moriguchi, M., et al. (1999) Influence of genotypes at SAA1 and SAA2 loci on the development and the length of latent period of secondary AA-amyloidosis in patients with rheumatoid arthritis. Hum. Genet. 105, 360-366). According to the method, two SNPs on the same chromosome located at two different sites are amplified together in a single molecule. Thus, experimental data obtained by the method enables determination of the diplotype configuration for each subject. Some related studies using the data for the SAA gene have been reported, but no haplotype analysis had been carried out in the previous studies (Moriguchi, M., et al., 1999; Moriguchi, M., et al. (2001) A novel single-nucleotide polymorphism at the 5'-flanking region of SAA1 associated with risk of type AA Amyloidosis secondary to rheumatoid arthritis. Arthritis Rheum. 44, 1266-1272). The data obtained for the six SNP loci were analyzed by only the EM-based computation.

The haplotype frequencies thus determined were completely different from those computed on the assumption that no linkage disequilibrium exists in all three distinct Japanese groups. Table 4 shows a comparison of the numbers and frequencies of haplotypes in the region of the SAA gene between those computed from the data obtained in *in-vitro* experiments and those obtained according to the EM algorithm. Table 5 shows the numbers of subjects having each diplotype configuration in the region of the SAA gene determined physically (left) and those computed according to the EM algorithm (right).

Table 4

| Group[a] | haplotype[b] | number of haplotypes | haplotype frequency | | |
|---|---|---|---|---|---|
| | | | haplotype frequency determined in the in-vitro experiments | haplotype frequency calculated according to the EM algorithm | haplotype frequency calculated on the assumption that SNPs at different sites are independent of one another [c] |
| amyloid | CC | 51 | 0.580 | 0.580 | 0.614 |
| | CT | 27 | 0.307 | 0.307 | 0.272 |
| | TC | 10 | 0.114 | 0.114 | 0.079 |
| | TT | 0 | 0 | 0 | 0.035 |
| RA | CT | 41 | 0.373 | 0.373 | 0.241 |
| | TC | 39 | 0.355 | 0.355 | 0.222 |
| | CC | 30 | 0.273 | 0.273 | 0.405 |
| | TT | 0 | 0 | 0 | 0.132 |
| control | CC | 45 | 0.388 | 0.388 | 0.482 |
| | TC | 36 | 0.310 | 0.310 | 0.217 |
| | CT | 35 | 0.302 | 0.302 | 0.208 |

(continued)

| Group[a] | haplotype[b] | number of haplotypes | haplotype frequency | | |
|---|---|---|---|---|---|
| | | | haplotype frequency determined in the in-vitro experiments | haplotype frequency calculated according to the EM algorithm | haplotype frequency calculated on the assumption that SNPs at different sites are independent of one another [c] |
| | TT | 0 | 0 | 0 | 0.094 |

[a]Amyloid: RA patients with secondary AA amyloidosis, RA: patients with early RA, control: control subjects.

[b]Haplotype containing the SNP loci, saas-5 and saas-6, shown in Fig. 5.

[c]Haplotype frequency computed from the SNP frequency on the assumption that SNPs at different sites are independent of one another.

Table 5

| amyloid[a] | haplotype[b] | CC | CT | TC |
|---|---|---|---|---|
| | CC | 16/16 | 15/15 | 4/4 |
| | CT | - | 4/4 | 4/4 |
| | TC | — | — | 1/1 |
| RA[a] | haplotype[b] | CC | CT | TC |
| | CC | 2/2 | 10/10 | 16/16 |
| | CT | - | 9/9 | 13/13 |
| | TC | - | - | 5/5 |
| control[a] | haplotype[b] | CC | CT | TC |
| | CC | 7/7 | 17/17 | 14/14 |
| | CT | - | 4/4 | 10/10 |
| | TC | - | - | 6/6 |

[a]Amyloid: RA patients with secondary AA amyloidosis, RA: patients with early RA, control: control subjects.

[b]Haplotype containing the SNP loci, saas-5 and saas-6, shown in Fig. 5.

Example 5: Computation of haplotype frequencies using the EM algorithm and diplotype configuration of saa1 determined for each subject

The haplotype frequencies were computed using the same data of saa1 as in Example 4. In this computation, only genotypic data for two SNP loci, saas-5 and saas-6, were included in the input file. Each of the data from the three groups (i.e., amyloid group, RA group and control group) was independently applied for the analysis. According to the data from the output file, the diplotype distribution for each subject was found to be concentrated in a single diplotype configuration. In all the three groups, the diplotype configuration agreed with that physically determined in *in-vitro* experiments. The diplotype configuration of each of the subjects is not shown, but among the subjects in the three groups, no subject had two or more diplotype configurations nor showed concentration in a diplotype configuration different from the physically determined one (Table 5).

As the conclusion, in all the three groups, the expected value of each haplotype frequency was identical to the percentage of the haplotypes in the sample (Table 4).

Example 6: Calculation for saa1 using combined data sets

When the data for all the groups in combination were executed in the program, the diplotype distribution of each subject concentrated in a single diplotype configuration, which diplotype configuration was identical to that physically determined in *in-vitro* experiments. Table 6 shows the numbers of subjects who have respective diplotype configurations for regions of the two SNP loci of the SAA gene in all the combined groups, those physically determined (left) and those computed according to the EM algorithm (right). As the conclusion, the computed haplotype frequencies were identical to those computed from the data obtained in *in-vitro* experiments.

Table 6

| haplotype[a,b] | CC | CT | TC |
|---|---|---|---|
| CC | 25/25 | 42/42 | 34/34 |
| CT | - | 17/17 | 27/27 |
| TC | - | - | 12/12 |

[a]The data from the three distinct Japanese groups (RA patients with secondary AA amyloidosis, patients with early RA, and control patients) were combined and applied for ldsupport software. None of them had diplotype distribution concentrated in two or more diplotype configurations.

[b]Haplotype containing the SNP loci, saas-5 and saas-6, shown in Fig. 5.

Example 7: Computation of haplotype frequencies for the six SNP loci of the SAA gene

The genotypes of six SNP loci, saas-1, -2, -3, -4, - 5 and -6, in the SAA gene of the amyloid, RA or control groups were executed in the LDSUPPORT program. In this case, the SNP loci are located in a wider region within the SAA gene (Fig. 5). As shown in Fig. 5, saas-1 is located within saa2, but the remaining loci exist within saa1. On the assumption that no linkage disequilibrium exists, the computed frequency was completely different from the computed haplotype frequency. Table 7 shows the haplotype frequencies for the six SNP loci within the SAA gene in control subjects that were computed according to the EM algorithm.

Table 7

| haplotype[a] | amyloid[b] | | RA[b] | | control [b] | |
|---|---|---|---|---|---|---|
| | Example1[c] | Example2[c] | Example1[c] | Example2[c] | Example1[c] | Example2[c] |
| ACTGCC | 61.1% | 28.0% | 26.2% | 7.4% | 38.7% | 13.2% |
| AGCGCT | 19.1% | 1.7% | 24.3% | 4.4% | 18.2% | 2.5% |
| GCCGTC | 3.5% | 0.2% | 1.7% | 0.9% | 4.5% | 1.1% |
| GCCGTT | 3.2% | 0.1% | 0.0% | 0.5% | 0.0% | 0.5% |
| GCTGCT | 2.5% | 1.6% | 2.6% | 0.5% | 3.4% | 0.8% |
| ACCGTC | 2.4% | 1.8% | 30.1% | 8.3% | 25.7% | 7.6% |
| AGCACT | 1.3% | 0.0% | 2.8% | 0.4% | 0.9% | 0.1% |
| ACTGCT | 1.3% | 12.4% | 1.9% | 4.4% | 1.8% | 5.7% |
| GGCGCT | 1.2% | 0.2% | 2.1% | 0.5% | 2.4% | 0.4% |
| ACTGTC | 1.1% | 3.6% | 1.8% | 4.0% | 0.0% | 5.9% |
| ACCGCC | 1.1% | 13.8% | 0.9% | 15.1% | 0.0% | 16.8% |
| ACCGTT | 1.1% | 0.8% | 0.0% | 4.9% | 0.0% | 3.3% |
| GCTACT | 1.0% | 0.0% | 0.0% | 0.0% | 0.0% | 0.0% |
| GGCACT | 0.0% | 0.0% | 3.5% | 0.0% | 2.6% | 0.0% |
| ACCATC | 0.0% | 0.0% | 1.8% | 0.7% | 0.0% | 0.0% |
| GCTGCC | 0.0% | 3.6% | 0.1% | 0.8% | 0.0% | 2.0% |

(continued)

| haplotype[a] | amyloid[b] | | RA[b] | | control [b] | |
|---|---|---|---|---|---|---|
| | Example1[c] | Example2[c] | Example1[c] | Example2[c] | Example1[c] | Example2[c] |
| AGCGCC | 0.0% | 3.8% | 0.0% | 7.4% | 0.9% | 5.9% |
| AGCGTT | 0.0% | 0.0% | 0.0% | 2.4% | 0.9% | 1.1% |

[a]Haplotype containing the SNP loci, saas-1, -2, -3, -4, -5 and saas-6, shown in Fig. 5.

[b]Amyloid: RA patients with secondary AA amyloidosis, RA: patients with early RA, control: control subjects.

[c]Example 1: haplotype frequencies for the six SNP loci estimated by a method based on the EM algorithm; Example 2: haplotype frequencies computed from the frequencies of SNPs on the assumption that the six SNP loci are independent of each other.

Tables 8 and 9 show diplotype distribution for the six SNP loci in the SAA gene of each subject in each of the groups. 42 of 43 subjects (97.7%) in the amyloid group (panel A) , RA group (panel B) and control group (panel C) exhibited diplotype distribution that is concentrated in a single diplotype configuration (Tables 8 and 9). The numbers of subjects who have diplotype distributions that are not concentrated in single configuration and respective distributions are shown under each panel of Tables 8 and 9. 1/43 (2.3%), 2/55 (3.6%) and 2/58 (3.4%) of the diplotype distribution for each subject were not concentrated in single diplotype configuration in the amyloid group, RA group and control group, respectively. Table 10 shows the numbers of subjects who have respective diplotype configurations in all of the groups computed according to the EM algorithm and the numbers of subjects who have diplotype distributions that concentrates in two or more diplotype configurations for the six gene loci in the SAA gene.

Table 8

(panel A)

| haplotype[a] | 0 | 1 | 2 | 3 | 4 | 5 | 6 | 7 | 8 | 9 | 10 | 11 | 12 |
|---|---|---|---|---|---|---|---|---|---|---|---|---|---|
| 0 | 15 | 12 | 3 | 3 | 1 | 1 | 1 | 0 | 1 | 0 | 1 | 1 | 0 |
| 1 | — | 1 | 0 | 0 | 1 | 0 | 0 | 1 | 0 | 0 | 0 | 0 | 0 |
| 2 | — | — | 0 | 0 | 0 | 0 | 0 | 0 | 0 | 0 | 0 | 0 | 0 |
| 3 | — | — | — | 0 | 0 | 0 | 0 | 0 | 0 | 0 | 0 | 0 | 0 |
| 4 | — | — | — | — | 0 | 0 | 0 | 0 | 0 | 0 | 0 | 0 | 0 |
| 5 | — | — | — | — | — | 0 | 0 | 0 | 0 | 1 | 0 | 0 | 0 |
| 6 | — | — | — | — | — | — | 0 | 0 | 0 | 0 | 0 | 0 | 0 |
| 7 | — | — | — | — | — | — | — | 0 | 0 | 0 | 0 | 0 | 0 |
| 8 | — | — | — | — | — | — | — | — | 0 | 0 | 0 | 0 | 0 |
| 9 | — | — | — | — | — | — | — | — | — | 0 | 0 | 0 | 0 |
| 10 | — | — | — | — | — | — | — | — | — | — | 0 | 0 | 0 |
| 11 | — | — | — | — | — | — | — | — | — | — | — | 0 | 0 |
| 12 | — | — | — | — | — | — | — | — | — | — | — | — | 0 |

| number of subjects[b] | diplotype configuration (probability) |
|---|---|
| 1 | 1/12 (0.97), 4/6 (0.03) |

(panel B)

| haplotype[a] | 5 | 0 | 1 | 13 | 6 | 4 | 8 | 7 | 14 | 9 | 2 | 10 | 15 |
|---|---|---|---|---|---|---|---|---|---|---|---|---|---|
| 5 | 4 | 13 | 6 | 1 | 0 | 2 | 0 | 1 | 1 | 0 | 0 | 0 | 0 |
| 0 | — | 2 | 6 | 1 | 0 | 0 | 1 | 1 | 0 | 2 | 1 | 0 | 0 |
| 1 | — | — | 5 | 1 | 1 | 1 | 0 | 0 | 0 | 0 | 0 | 1 | 0 |
| 13 | — | — | — | 0 | 0 | 0 | 0 | 0 | 1 | 0 | 0 | 0 | 0 |
| 6 | — | — | — | — | 1 | 0 | 0 | 0 | 0 | 0 | 0 | 0 | 0 |
| 4 | — | — | — | — | — | 0 | 0 | 0 | 0 | 0 | 0 | 0 | 0 |
| 8 | — | — | — | — | — | — | 0 | 0 | 0 | 0 | 0 | 0 | 0 |
| 7 | — | — | — | — | — | — | — | 0 | 0 | 0 | 0 | 0 | 0 |
| 14 | — | — | — | — | — | — | — | — | 0 | 0 | 0 | 0 | 0 |
| 9 | — | — | — | — | — | — | — | — | — | 0 | 0 | 0 | 0 |
| 2 | — | — | — | — | — | — | — | — | — | — | 0 | 0 | 0 |
| 10 | — | — | — | — | — | — | — | — | — | — | — | 0 | 0 |
| 15 | — | — | — | — | — | — | — | — | — | — | — | — | 0 |

| number of subjects[b] | diplotype configuration (probability) |
|---|---|
| 2 | 5/8 (0.72), 1/2 (0.28) |

Table 9

(panel **C**)

| haplotype[a] | 0 | 5 | 1 | 2 | 4 | 13 | 8 | 7 | 16 | 6 | 17 |
|---|---|---|---|---|---|---|---|---|---|---|---|
| 0 | 6 | 12 | 10 | 2 | 3 | 2 | 1 | 1 | 1 | 0 | 1 |
| 5 | — | 4 | 5 | 2 | 1 | 0 | 0 | 1 | 0 | 0 | 0 |
| 1 | — | — | 2 | 0 | 0 | 0 | 1 | 0 | 0 | 0 | 0 |
| 2 | — | — | — | 0 | 0 | 0 | 0 | 0 | 0 | 0 | 0 |
| 4 | — | — | — | — | 0 | 0 | 0 | 0 | 0 | 0 | 0 |
| 13 | — | — | — | — | — | 0 | 0 | 0 | 0 | 1 | 0 |
| 8 | — | — | — | — | — | — | 0 | 0 | 0 | 0 | 0 |
| 7 | — | — | — | — | — | — | — | 0 | 0 | 0 | 0 |
| 16 | — | — | — | — | — | — | — | — | 0 | 0 | 0 |
| 6 | — | — | — | — | — | — | — | — | — | 0 | 0 |
| 17 | — | — | — | — | — | — | — | — | — | — | 0 |

| number of subjects[b] | diplotype configuration (probability) |
|---|---|
| 2 | 1/2 (0.63), 5/8 (0.37) |

Table 10

| haplotype[a] | 0 | 1 | 2 | 3 | 4 | 5 | 6 | 7 | 8 | 9 | 10 | 11 | 12 |
|---|---|---|---|---|---|---|---|---|---|---|---|---|---|
| 0 | 23[b] | 26 | 28 | 4 | 6 | 3 | 2 | 1 | 3 | 2 | 1 | 0 | 1 |
| 1 | — | 8 | 11 | 6 | 2 | 0 | 3 | 0 | 0 | 1 | 0 | 1 | 0 |
| 2 | — | — | 8 | 2 | 0 | 1 | 1 | 1 | 1 | 1 | 1 | 0 | 0 |
| 3 | — | — | — | 0 | 0 | 0 | 0 | 0 | 0 | 0 | 0 | 0 | 0 |
| 4 | — | — | — | — | 0 | 0 | 0 | 0 | 0 | 0 | 0 | 0 | 0 |
| 5 | — | — | — | — | — | 0 | 0 | 1 | 0 | 0 | 0 | 1 | 0 |
| 6 | — | — | — | — | — | — | 0 | 0 | 0 | 0 | 0 | 0 | 0 |
| 7 | — | — | — | — | — | — | — | 1 | 0 | 0 | 0 | 0 | 0 |
| 8 | — | — | — | — | — | — | — | — | 0 | 0 | 0 | 0 | 0 |
| 9 | — | — | — | — | — | — | — | — | — | 0 | 0 | 0 | 0 |
| 10 | — | — | — | — | — | — | — | — | — | — | 0 | 0 | 0 |
| 11 | — | — | — | — | — | — | — | — | — | — | — | 0 | 0 |
| 12 | — | — | — | — | — | — | — | — | — | — | — | — | 0 |

| number of subjects[c] | diplotype configuration (probability) |
|---|---|
| 1 | 1/5 (0.98), 4/7 (0.02) |
| 4 | 2/4 (0.80), 1/8 (0.20) |
| 1 | 3/7 (0.64), 5/6 (0.36) |

ᵃHaplotype containing the SNP loci, saas-1, -2, -3, -4, -5 and saas-6, shown in Fig. 5. The haplotypes are numbered as follows: 0: ACTGCC, 1:ACCGTC, 2:AGCGCT, 3:GCTGTC, 4:GCCGTC, 5:GGCACT, 6:ACTGCT, 7:AGCACT, 8:GGCGCT, 9:ACTGTC, 10:ACCGCC, 11:ACCATC, 12:AGCGCC.

ᵇThe data from the three different Japanese groups (RA patients with secondary AA amyloidosis, patients with early RA and control patients) were combined and applied to the ldsupport software.

ᶜThe number of subjects having diplotype distributions concentrated in two or more diplotype configurations.

Industrial Applicability

The present invention provides a method for inferring diplotype configuration from genotypic information of an individual without family data. The diplotype configurations inferred by the present method are highly reliable. According to the present method, the diplotype configuration can be effectively inferred even when no family data is available. Using the diplotype configuration inferred by the present method, the phenotype of a subject can be conveniently and rapidly identified. Furthermore, the present invention makes it possible to compute the probability of a specific drug showing effectiveness in an individual, the probability that a specific drug causes side effect on an individual, or the probability that an individual develops a specific disease in the future or is affected with the disease. The probabilities thus computed serve as important factors for judging the possible effect or risk of side effects of a drug administered to an individual. These probabilities can also be used to determine whether a specific drug should be administered or the dose of a drug is appropriate for the subject. Data of haplotype frequencies in a population, data of diplotype distributions and databases constructed based on the data of diplotype configurations (which can be obtained by the present method) sever as important information sources to make such judgments. Thus, the present invention serves as a basic technology for personalized medical treatment (tailor-made medical treatment).

**Claims**

1.  A method for computing diplotype distribution for each subject in a population without family data, which comprises the step of computing the diplotype distribution for each subject based on previously estimated or determined haplotype frequencies in the population and the genotypic information of each subject.

2.  The method according to claim 1, wherein the haplotype frequencies are computed in the population from genotypic information of each subject in the population just prior the step of computing the diplotype distribution.

3.  The method according to claim 2, wherein the computation of haplotype frequencies is performed using the EM algorithm.

4.  The method according to claim 1 or 2, wherein the computation of diplotype distribution is performed using Bayes' theorem or computation of posterior probability.

5. The method according to any one of claims 1 to 4, wherein the genotypic information is a polymorphism selected from the group consisting of single nucleotide polymorphism (SNP), microsatellite polymorphism and insertion/deletion polymorphism.

6. A method for computing the probability of a subject to exhibit a specific phenotype, which comprises the steps of:

   (a) computing diplotype distribution for the subject without family data using the method according to any one of claims 1 to 5; and
   (b) computing the probability that the subject exhibits the specific phenotype from the diplotype distribution computed in step (a) based on previously estimated or determined probability that an individual having a specific diplotype configuration exhibits the specific phenotype.

7. A method for computing the probability that a specific drug is effective or causes side effect in a subject, which comprises the steps of:

   (a) computing diplotype distribution for the subject without family data using the method according to any one of claims 1 to 5; and
   (b) computing the probability that the specific drug is effective or causes side effect in the subject from the diplotype distribution computed in step (a) based on previously estimated or determined probability that the specific drug is effective or causes side effect in an individual having a specific diplotype configuration.

8. A method for computing the probability that a subject develops or is affected with a disease, which comprises the steps of:

   (a) computing diplotype distribution for the subject without family data using the method according to any one of claims 1 to 5; and
   (b) computing the probability that the subject develops or is affected with the disease from the diplotype distribution computed in step (a) based on previously estimated or determined probability that an individual having a specific diplotype develops or is affected with the disease.

9. A program to compute diplotype distribution for each subject in a population from genotypic information of each subject, which comprises code means which when executed on a computer perform the step of the method according to claim 1.

10. The program of claim 9, wherein the haplotype frequencies are computed in the population from genotypic information of each subject in the population just prior the step of computing the diplotype distribution.

11. A program to compute the probability of a subject to exhibit a specific phenotype, which comprises code means which when executed on a computer perform the steps (a) and (b) of the method according to claim 6.

12. A program to compute the probability that a specific drug is effective or causes side effect in a subject, which comprises code means which when executed on a computer perform the steps (a) and (b) of the method according to claim 7.

13. A program to compute the probability that a subject develops or is affected with a disease, which comprises code means which when executed on a computer perform the steps (a) and (b) of the method according to claim 8.

14. A computer-readable recording medium wherein the program according to any one of claims 9 to 13 is stored.

15. A device to compute diplotype distribution for each subject in a population without family data, which consists of:

   (a) an input means for entering genotypic information of each subject in the population;
   (b) a means for calculating haplotype frequencies from genotypic information of each subject in the population;
   (c) a means for calculating diplotype distribution for each subject based on the haplotype frequencies in the population and the genotypic information of each subject; and
   (d) a display means to display the calculated diplotype distribution.

16. The device of claim 15, wherein step (b) is replaced with:

(b') an input means to enter previously estimated or determined haplotype frequencies of a population.

**17.** A method for selecting a medical treatment appropriate for a subject, which comprises the steps of:

(a) computing the probability that a specific drug is effective or causes side effect in the subject by the method according to claim 7; and
(b) determining whether to administer the drug, or the dose of the drug based on the probability computed in step (a).

**18.** A method for selecting a medical treatment appropriate for a subject, which comprises the steps of:

(a) computing the probability that the subject develops or is affected with a specific disease by the method according to claim 8; and
(b) determining whether to perform treatment or prevention against the disease, or selecting a method for the treatment or prevention based on the probability computed in step (a).

**Patentansprüche**

**1.** Verfahren zum Berechnen einer Diplotyp-Verteilung für jede Person in einer Population ohne Familiendaten, das den Schritt umfasst, die Diplotyp-Verteilung für jede Person auf der Grundlage von zuvor abgeschätzten oder ermittelten Haplotyp-Frequenzen in der Population und der genotypischen Information jeder Person zu berechnen.

**2.** Verfahren nach Anspruch 1, bei dem die Haplotyp-Frequenzen in der Population ausgehend von genotypischer Information jeder Person in der Population kurz vor dem Schritt berechnet werden, die Diplotyp-Verteilung zu berechnen.

**3.** Verfahren nach Anspruch 2, bei dem die Berechnung von Haplotyp-Frequenzen unter Verwendung des EM-Algorithmus durchgeführt wird.

**4.** Verfahren nach Anspruch 1 oder 2, bei dem die Berechnung der Diplotyp-Verteilung unter Verwendung des Bayes'schen Satzes oder Berechnung einer Posteriori-Wahrscheinlichkeit durchgeführt wird.

**5.** Verfahren nach einem der Ansprüche 1 bis 4, bei dem die genotypische Information ein Polymorphismus ist, der aus der Gruppe ausgewählt ist, die aus einem Einzelnukleotidpolymorphismus (SNP), einem Mikrosatelliten-Polymorphismus und einem Insertions-/Deletions-Polymorphismus besteht.

**6.** Verfahren zum Berechnen der Wahrscheinlichkeit, dass eine Person einen speziellen Phänotyp an den Tag legt, das die Schritte umfasst:

(a) Berechnen einer Diplotyp-Verteilung für die Person ohne Familiendaten unter Verwendung des Verfahrens gemäß einem der Ansprüche 1 bis 5; und
(b) Berechnen der Wahrscheinlichkeit, dass die Person den speziellen Phänotyp an den Tag legt, aus der in Schritt (a) berechneten Diplotyp-Vcrteilung auf der Grundlage einer zuvor abgeschätzten oder ermittelten Wahrscheinlichkeit, dass ein Individuum mit einer speziellen Diplotyp-Struktur, den speziellen Phänotyp an den Tag legt.

**7.** Verfahren zum Berechnen der Wahrscheinlichkeit, dass ein spezielles Medikament bei einer Person wirksam ist oder eine Nebenwirkung verursacht, das die Schritte umfasst:

(a) Berechnen einer Diplotyp-Verteilung für die Person ohne Familiendaten unter Verwendung des Verfahrens gemäß einem der Ansprüche 1 bis 5; und
(b) Berechnen der Wahrscheinlichkeit, dass das spezielle Medikament bei der Person wirksam ist oder eine Nebenwirkung verursacht, ausgehend von der in Schritt (a) berechneten Diplotyp-Verteilung auf der Grundlage einer zuvor abgeschätzten oder ermittelten Wahrscheinlichkeit, dass das spezielle Medikament bei einem Individuum mit einer speziellen Diplotyp-Struktur wirksam ist oder eine Nebenwirkung verursacht.

**8.** Verfahren zum Berechnen der Wahrscheinlichkeit, dass eine Person sich eine Krankheit zuzieht oder von dieser befallen wird, das die Schritte umfasst:

(a) Berechnen einer Diplotyp-Verteilung für die Person ohne Familiendaten unter Verwendung des Verfahrens gemäß einem der Ansprüche 1 bis 5; und

(b) Berechnen der Wahrscheinlichkeit, dass die Person sich die Krankheit zuzieht oder von dieser befallen wird, ausgehend von der in Schritt (a) berechneten Diplotyp-Verteilung auf der Grundlage einer zuvor abgeschätzten oder ermittelten Wahrscheinlichkeit, dass ein Individuum mit einem speziellen Diplotyp sich die Krankheit zuzieht oder von dieser befallen wird.

9. Programm, um eine Diplotyp-Verteilung für jede Person in einer Population ausgehend von genotypischer Information jeder Person zu berechnen, das Codemittel umfasst, die, wenn sie auf einem Computer ausgeführt werden, die Schritte gemäß Anspruch 1 durchführen.

10. Programm nach Anspruch 9, bei dem die Haplotyp-Frequenzen in der Population ausgehend von genotypischer Information jeder Person in der Population kurz vor dem Schritt berechnet werden, die Diplotyp-Verteilung zu berechnen.

11. Programm, um die Wahrscheinlichkeit einer Person zu berechnen, einen speziellen Phänotyp an den Tag zu legen, das Codemittel umfasst, die, wenn sie auf einem Computer ausgeführt werden, die Schritte (a) und (b) des Verfahrens gemäß Anspruch 6 ausführen.

12. Programm, um die Wahrscheinlichkeit zu berechnen, dass ein spezielles Medikament bei einer Person wirksam ist oder eine Nebenwirkung verursacht, das Codemittel umfasst, die, wenn sie auf einem Computer ausgeführt werden, die Schritte (a) und (b) des Verfahrens gemäß Anspruch 7 durchführen.

13. Programm, um die Wahrscheinlichkeit zu berechnen, dass eine Person sich eine Krankheit zuzieht oder von dieser befallen wird, das Codemittel umfasst, die, wenn sie auf einem Computer ausgeführt werden, die Schritte (a) und (b) des Verfahrens gemäß Anspruch 8 durchführen.

14. Computerlesbares Aufzeichnungsmedium, auf dem das Programm gemäß einem der Ansprüche 9 bis 13 gespeichert ist.

15. Vorrichtung, um eine Diplotyp-Verteilung für jede Person in einer Population ohne Familiendaten zu berechnen, die besteht aus:

(a) einer Eingabeeinrichtung, um genotypische Information jeder Person in der Population einzugeben;
(b) einer Einrichtung, um Haplotyp-Frequenzen ausgehend von genotypischer Information jeder Person in der Population zu berechnen;
(c) einer Einrichtung, um eine Diplotyp-Verteilung für jede Person auf der Grundlage der Haplotyp-Frequenzen in der Population und der genotypischen Information jeder Person zu berechnen; und
(d) einer Wiedergabeeinrichtung, um die berechnete Diplotyp-Verteilung wiederzugeben.

16. Vorrichtung nach Anspruch 15, bei der Schritt (b) ersetzt ist durch:

(b') eine Eingabeeinrichtung, um zuvor abgeschätzte oder ermittelte Haplotyp-Frequenzen einer Population einzugeben.

17. Verfahren, um eine für eine Person geeignete, medizinische Behandlung zu wählen, das die Schritte umfasst:

(a) Berechnen der Wahrscheinlichkeit, dass ein spezielles Medikament bei einer Person wirksam ist oder eine Nebenwirkung verursacht, mittels des Verfahrens gemäß Anspruch 7; und
(b) Festlegen, ob das Medikament oder die Dosis des Medikaments zu verabreichen ist, auf der Grundlage der in Schritt (a) berechneten Wahrscheinlichkeit.

18. Verfahren, um eine für eine Person geeignete, medizinische Behandlung zu wählen, das die Schritte umfasst:

(a) Berechnen der Wahrscheinlichkeit, dass die Person sich eine spezielle Krankheit zuzieht oder von dieser betroffen wird, mittels des Verfahrens gemäß Anspruch 8; und

(b) Festlegen, ob eine Behandlung der Krankheit oder Prävention gegen diese durchzuführen ist, oder Wählen eines Verfahrens für die Behandlung oder Prävention auf der Grundlage der in Schritt (a) berechneten Wahrscheinlichkeit.

**Revendications**

1. Procédé pour le calcul de la distribution de diplotypes pour chaque sujet dans une population sans données familiales, comprenant l'étape de calcul de la distribution de diplotypes pour chaque sujet, basé sur des fréquences d'haplotypes déterminées ou évaluées précédemment dans la population et l'information génotypique de chaque sujet.

2. Procédé selon la revendication 1, dans lequel les fréquences d'haplotypes sont calculées dans la population à partir d'information génotypique de chaque sujet dans la population juste avant l'étape de calcul de la distribution de diplotypes.

3. Procédé selon la revendication 2, dans lequel le calcul des fréquences d'haplotypes est effectué à l'aide de l'algorithme EM.

4. Procédé selon la revendication 1 ou 2, dans lequel le calcul de la distribution de diplotypes est effectué à l'aide du théorème de Bayes ou du calcul de la probabilité postérieure.

5. Procédé selon l'une quelconque des revendications 1 à 4, dans lequel l'information génotypique est un polymorphisme choisi dans le groupe constitué par le polymorphisme d'un seul nucléotide (SNP), le polymorphisme de microsatellites et le polymorphisme d'insertion/délétion.

6. Procédé pour le calcul de la probabilité qu'un sujet présente un phénotype particulier, comprenant les étapes suivantes :

   (a) calcul de la distribution de diplotypes pour le sujet sans données familiales, à l'aide du procédé selon l'une quelconque des revendications 1 à 5 ; et
   (b) calcul de la probabilité que le sujet présente le phénotype particulier d'après la distribution de diplotypes calculée dans l'étape (a), basé sur la probabilité évaluée ou déterminée précédemment qu'un individu ayant une configuration particulière de diplotypes présente le phénotype particulier.

7. Procédé pour le calcul de la probabilité qu'un médicament particulier soit efficace ou provoque un effet secondaire chez un sujet, comprenant les étapes suivantes :

   (a) calcul de la distribution de diplotypes pour le sujet sans données familiales à l'aide du procédé selon l'une quelconque des revendications 1 à 5 ; et
   (b) calcul de la probabilité que le médicament particulier soit efficace ou provoque un effet secondaire chez le sujet d'après la distribution de diplotypes calculée dans l'étape (a), basé sur la probabilité évaluée ou déterminée précédemment que le médicament particulier soit efficace ou provoque un effet secondaire chez un individu ayant une configuration particulière de diplotypes.

8. Procédé pour le calcul de la probabilité qu'un sujet développe ou contracte une maladie, comprenant les étapes suivantes :

   (a) calcul de la distribution de diplotypes pour le sujet sans données familiales à l'aide du procédé selon l'une quelconque des revendications 1 à 5 ; et
   (b) calcul de la probabilité que le sujet développe ou contracte une maladie d'après la distribution de diplotypes calculée dans l'étape (a), sur la base de la probabilité évaluée ou déterminée précédemment qu'un individu ayant un diplotype particulier développe ou contracte la maladie.

9. Programme pour le calcul de la distribution de diplotypes pour chaque sujet dans une population à partir de l'information génétique de chaque sujet, comprenant des moyens de codage qui lorsqu'ils sont exécutés sur un ordinateur effectuent l'étape du procédé selon la revendication 1.

10. Programme selon la revendication 1, dans lequel les fréquences d'haplotypes sont calculées dans la population à partir d'information génotypique de chaque sujet dans la population juste avant l'étape de calcul de la distribution de diplotypes.

11. Programme pour le calcul de la probabilité qu'un sujet présente un phénotype particulier, comprenant des moyens de codage qui, lorsqu'ils sont exécutés sur un ordinateur, effectuent les étapes (a) et (b) du procédé selon la revendication 6.

12. Programme pour le calcul de la probabilité qu'un médicament particulier soit efficace ou provoque un effet secondaire chez un sujet, comprenant des moyens de codage qui, lorsqu'ils sont exécutés sur un ordinateur, effectuent les étapes (a) et (b) du procédé selon la revendication 7.

13. Programme pour le calcul de la probabilité qu'un sujet développe ou contracte une maladie, comprenant des moyens de codage qui, lorsqu'ils sont exécutés sur un ordinateur, effectuent les étapes (a) et (b) du procédé selon la revendication 6.

14. Support d'enregistrement lisible par ordinateur, sur lequel est mémorisé le programme selon l'une quelconque des revendications 9 à 13.

15. Dispositif pour le calcul de la distribution de diplotypes pour chaque sujet dans une population sans données familiales, consistant en :

    (a) un moyen de saisie pour l'entrée de l'information génétique de chaque sujet dans la population ;
    (b) un moyen de calcul des fréquences d'haplotypes à partir de l'information génétique de chaque sujet dans la population ;
    (c) un moyen de calcul de la distribution de diplotypes pour chaque sujet, basé sur les fréquences d'haplotypes dans la population et l'information génotypique de chaque sujet ;
    (d) un moyen d'affichage pour afficher la distribution calculée de diplotypes.

16. Dispositif selon la revendication 15, dans lequel l'étape (b) est remplacée par :

    (b') un moyen de saisie pour entrer des fréquences précédemment évaluées ou déterminées d'haplotypes d'une population.

17. Procédé pour la sélection d'un traitement médical approprié pour un sujet, comprenant les étapes consistant à :

    (a) calculer la probabilité qu'un médicament particulier soit efficace ou provoque un effet secondaire chez le sujet par le procédé selon la revendication 7 ; et
    (b) déterminer s'il convient d'administrer le médicament ou la dose sur la base de la probabilité calculée dans l'étape (a) ;

18. Procédé pour le choix d'un traitement médical approprié pour un sujet, comprenant les étapes consistant à :

    (a) calculer la probabilité que le sujet développe ou contracte une maladie particulière, par le procédé selon la revendication 8 ; et
    (b) déterminer s'il convient d'effectuer un traitement ou une prévention de la maladie, ou choisir une méthode de traitement ou de prévention sur la base de la probabilité calculée dans l'étape (a).

Fig. 1

TEMPORARY
STOREGE MEANS **4**

CENTRAL PROCESSING
UNIT (CPU) **5**

**3**

INPUT MEANS **1**

**6**

DISPLAY MEANS **2**

**61**
HAPLOTYPE FREQUENCY COMPUTATION
PROGRAM

**62**
DIPLOTYPE DISTRIBUTION COMPUTATION
PROGRAM

**63**
COMPUTATION PROGRAM FOR
CALCULATING PROBABILITY THAT A
SUBJECT EXHIBITS A SPECIFIC PHENOTYPE

**64**
COMPUTATION PROGRAM FOR THE
PROBABILITY THAT IN A SUBJECT
AN EFFECT OR SIDE EFFECT IS
GENERATED BY A SPECIFIC DRUG

**65**
COMPUTATION PROGRAM FOR
CALCULATING PROBABILITY THAT A
SUBJECT DEVELOPS OR IS AFFECTED
WITH A SPECIFIC DISEASE

**66**
CONTROL PROGRAM

FIG. 1

Fig. 2

FIG. 2

Fig. 3

FIG. 3

Fig. 4

FIG. 4

Fig. 5

FIG. 5

SEQUENCE LISTING

<110> HUBIT GENOMIX, INC.

<120> METHOD OF INFERRING DIPLOTYPE CONFIGURATION FROM THE GENOTYPE OF INDIVIDUALS

<130> H2-A0101P

<140>
<141>

<150> JP 2001-121641
<151> 2001-04-19

<160> 4

<170> PatentIn Ver. 2.0

<210> 1
<211> 21

<212> DNA
<213> Artificial Sequence

<220>
<223> Description of Artificial Sequence:Artificially
Synthesized Primer Sequence

<400> 1
taggaaccta gtaggtcctt a          21

<210> 2
<211> 22
<212> DNA
<213> Artificial Sequence

<220>
<223> Description of Artificial Sequence:Artificially Synthesized Primer Sequence

<400> 2
aaggaaatag ttgtagccac ta          22

<210> 3
<211> 19
<212> DNA
<213> Artificial Sequence

<220>
<223> Description of Artificial Sequence:Artificially Synthesized Primer Sequence

<400> 3
actgcctccg tgccatttg          19

<210> 4
<211> 19
<212> DNA
<213> Artificial Sequence

<220>
<223> Description of Artificial Sequence:Artificially Synthesized Primer Sequence

<400> 4
cagatccagt aagcctcac          19